# EUROPEAN PATENT SPECIFICATION

(11) **EP 0 974 672 B1**
(45) Date of publication and mention of the grant of the patent: **09.04.2003**
(21) Application number: 98202451.5
(22) Date of filing: 21.07.1998
(51) Int. Cl.: C12Q 1/68

(54) **Improved primers for AFLP amplification**
Verbesserte Primer zur AFLP Amplifizierung
Amorces ameliores pour l'amplification AFLP

(43) Date of publication of application: 26.01.2000
(73) Proprietor: KEYGENE N.V., 6700 AE Wageningen (NL)
(72) Inventor: Witsenboer, Hanneke, 6701 EC Wageningen (NL); Kuiper, Martin Tiemen Roelof, 3010 Kessel-Lo (BE)
(74) Representative: de Bruijn, Leendert C.

(56) References cited:
- EP-A- 0 534 858
- WO-A-96/11937
- GB-A- 2 295 228
- NICHOLS R ET AL: "A UNIVERSAL NUCLEOSIDE FOR USE AT AMBIGOUS SITES IN DNA PRIMERS" NATURE, vol. 369, no. 6480, 9 June 1994 (1994-06-09), page 492/493 XP000560346 ISSN: 0028-0836
- LOAKES D. ET AL.,: "3-Nitropyrrole and 5-nitroindole as universal bases in primers for DNA sequencing and PCR" NUCLEIC ACIDS RESEARCH, vol. 23, no. 13, - 1995 pages 2361-2366, XP002109690
- VOS P. ET AL.,: "AFLP: a new technique for DNA fingerprinting" NUCLEIC ACIDS RESEARCH, vol. 23, no. 21, - 1995 pages 4407-4414, XP002109691
- HIROKO FURUYAMA ET AL: "DNA SEQUENCING DIRECTLY FROM A MIXTURE USING TERMINAL-BASE- SELECTIVE PRIMERS" DNA RESEARCH, vol. 1, no. 5, 31 October 1994 (1994-10-31), pages 231-237, XP000609468 ISSN: 1340-2838
- JENSEN K K ET AL: "KINETICS FOR HYBRIDIZATION OF PEPTIDE NUCLEIC ACIDS (PNA) WITH DNA AND RNA STUDIED WITH THE BIACORE TECHNIQUE" BIOCHEMISTRY, vol. 36, 1 January 1997 (1997-01-01), pages 5072-5077, XP002062488 ISSN: 0006-2960
- MARTIN F.H.& CASTRO M.M.: "Base pairing involving deoxyinosine:implications for probe design" NUCLEIC ACIDS RESEARCH, vol. 13, no. 24, - 1985 pages 8927-8938, XP002109692

## Description

The present invention relates to improvements in DNA-amplification technology, in particular AFLP-technology. More specifically, the invention relates to the use of improved primers in AFLP that reduce the occurrence of so-called "mismatch priming". This increases the clarity and reliability of the generated fingerprint.

Selective restriction fragment amplification or AFLP is known, for instance from the European patent application 0 534 858 by applicant. In general, AFLP comprises the steps of:
(a) digesting a nucleic acid, in particular a DNA, with one or more specific restriction endonucleases, to fragment said DNA into a corresponding series of restriction fragments;
(b) ligating the restriction fragments thus obtained with at least one double-stranded synthetic oligonucleotide adapter, one end of which is compatible with one or both of the ends of the restriction fragments, to thereby produce tagged restriction fragments of the starting DNA;
(c) contacting said tagged restriction fragments under hybridizing conditions with at least one oligonucleotide primer;
(d) amplifying said tagged restriction fragment hybridized with said primers by PCR or a similar technique so as to cause further elongation of the hybridized primers along the restriction fragments of the starting DNA to which said primers hybridized; and
(e) identifying or recovering the amplified or elongated DNA fragment thus obtained.

The amplified DNA-fragments thus obtained can then be analysed and/or visualised, for instance by means of gel-electrophoresis. This provides a genetic fingerprint showing specific bands corresponding to the restriction fragments which have been linked to the adapter, have been recognized by the primer, and thus have been amplified during the amplification step. The fingerprint thus obtained provides information on the specific restriction site pattern of the starting DNA, and thus on the genetic make-up of the organism from which said DNA has been derived.

AFLP can therefore be used to identify said DNA, to analyse it for the the presence of specific restriction site patterns or restriction fragment length polymorfisms (RFLP's) corresponding to the presence of certain genes or genetic traits, or for similar purposes, for instance by comparing it to DNA-samples of known origin or restriction pattern, or data thereon.

As AFLP provides amplification of both strands of a double stranded starting DNA, AFLP advantageously allows for exponential amplification of the fragment, i.e. according to the series 2, 4, 8, 16, etc.. Also, AFLP requires no prior knowledge of the DNA sequence to be analysed, nor prior identification of suitable probes and/or the construction of a gene library from the starting DNA.

In AFLP, the primer used should be such that it recognizes and binds to the adapter, so as to serve as a "starting point" for the polymerase extension of the primer along the template. To this end, the primers usually have a nucleotide sequence that can hybridise with at least the sequence adjacent to the 3' end of the restriction fragment to be amplified (i.e. the sequence at the 5'-end of the adapter used).

Besides this so-called "constant region" of the primer sequence, the primers used in AFLP can also contain one or more further bases (called "selective bases") at the 3'-end of their sequence, for hybridisation with any complementary base or bases at the 3'-end of the adapter ligated restriction fragment. During the amplification step, of all the adapter ligated restiction fragments present in the mixture, only those fragments that contain bases complementary to the selective bases next to the ligated adapter (i.e. at the 3' ends of both strands of the double stranded fragment) will be amplified exponentially and therefore provide a band in the final fingerprint.

In this way, the use of these "selective" primers reduces the total amount of bands in the final fingerprint, thus making the fingerprint more clear and more specific. Also, the use of different selective primers will generally provide differing fingerprints, which can also be used as a tool for the purposes of identification or analysis.

These selective bases, which are not dependent upon the sequence of the adapter used and can generally be chosen either at random or by means of trial and error so as to provide the most informative fingerprint, form the so-called "variable region" of the primer. The number of selective bases is conventionally indicated by a plus-sign followed by a whole number: a "+*n*-primer" indicating a primer containing *n* selective bases.

In practice, AFLP amplifications with primers containing 3 or more selective bases are often carried out as multiple step procedures, in which the final amplification with the primer(s) with the desired number of selective bases is preceded by one or more so-called "pre-amplifications" with primers containing a fewer number of selective bases. For instance, +3 or +4 amplifications are often carried out as a two step amplification comprising a +1 (or sometimes a +2) preamplification followed by a final +3 or +4 amplification. The final amplification is then carried out on the amplified mixture obtained from the preamplification(s). In such a preamplification, the specific selective bases of the primers will as a rule correspond to the selective bases present in the corresponding positions of the final +n-primer.

In general, the use of a preamplification will greatly increase both the relative and absolute amount of suitable target templates, compared to the total number of adapter ligated restriction fragments present in the original starting mixture. A preamplification therefore provides for an increased availability of suitable target templates in the preamplified mixture (compared to the original starting mixture), so that during the final amplification, more primers can find the correct template.

For a further description of AFLP, its advantages, its embodiments, as well as the techniques, enzymes, adapters, primers and further compounds and tools used therein, reference is made to EP-0 534 858. Also, in the description hereinbelow, the definitions given in paragraph 5.1 of EP-0 534 858 will be used, unless indicated otherwise.

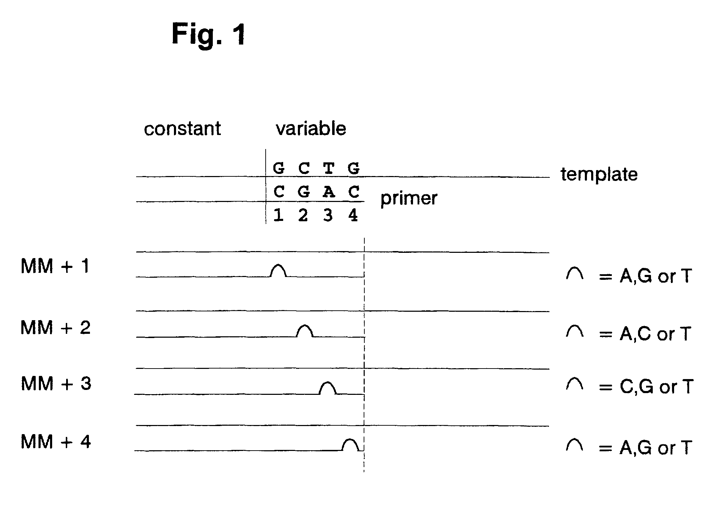

In principle, the primer sequence must be fully complementary to the corresponding sequence on the template. When this is not the case (i.e. when there is a sequence "mismatch" between the primer and the template) no extension of the primer along the template should take place (vide figure 1). Otherwise, fragments other than those desired or specifically intended (i.e. those bands which indicate the presence or absence of the genetic trait of interest, for instance by comparison with a a prior fingerprint or a database) may be amplified to a large or lesser extend and lead to contaminating bands (herein referred to as "mismatch bands") in the fingerprint.

This so-called "mismatch priming" can therefore reduce the clarity and/or reliability of the fingerprint obtained, in particular when such a mismatch takes place at or near the critical 3' end of the primer sequence, where primer extension is initiated. This can reduce the selectivity of the primer, as the amount of fragments with which the primer hybridises (and which thereby are amplified) is increased with these undesired fragments. This can particularly affect "analytical" AFLP techniques, in which the (repeated) choice of specific selective bases is often used to provide fingerprints which can be directly compared to known fingerprints or data (generally fingerprints or data obtained using the same protocol, restriction enzymes, primers, adapters, etc.).

Generally, the occurrance of a mismatch in the constant region of the primer sequence will be virtually impossible, as the constant region of the primer is chosen exactly to correspond to the (unique and known) adapter sequence.

In practice, although there is in principle no limit to the number of selective bases that can be used, it has been found that the occurrence of mismatch bands may increase when primers with 4 or more, and in particular 6 or more selective bases are used.

More generally, it has been found that when primers with 4 or more, and in particular with 6 or more, selective bases are used, there is no longer a suitable "balance" between the total number of primers used and the total number of intended target templates to be amplified/detected available. In a representative starting mixture of adapter ligated restriction fragments, there may be less than 10, or sometimes even only one or two of the correct templates present (among thousands of other fragments). This may not only mean that the amount of available template is too low in absolute terms, but also that the (excess of) primers will have difficulty in finding a correct template and may bind to other adapter ligated fragments, despite a sequence mismatch. They may even cause these undesired fragments to be amplified, leading to further mismatch bands. It has also been found that there is much more mispriming with +4 primers on +1+1- preamplification than on +1+2 preamplification.

In principle, the occurrence of such mismatches could be reduced or even prevented by using preamplifications, in which -for instance- the number of selective nucleosides in each primer is stepwise increased by one for each preamplification. However, carrying out such serial or stepwise pre-amplification(s) is very time consuming and highly impractical, so that most of the advantages of AFLP, in particular the rate and amount in which valuable results can be generated, would be lost.

However, using preamplifications in which the number of selective bases is stepwise increased by 3 would again lead to an increase in mismatch priming. This would also prevent "walking" along a restriction fragment, i.e. serial or stepwise amplifications using for instance +3, +6, +9, etc. primers, which could have some important practical applications.

In the art several publications have suggested the use of modified or alternative nucleosides. For instance, GB-A 2 295 228 describes the selective amplification of a subset of tagged restriction fragments wherein the restriction endonuclease restriction site is degenerated (contains unidentified bases) and wherein either the adaptor or the primer contains selective nucleotides to enable the level of mismatching to be reduced. Degenerated restriction endonuclease restriction sites are sites that have a degeneracy within the restriction site, whereby cleavage occurs within or outside the region of degeneracy or those whereby the DNA is cleaved at a position at a specific distance from a specific recognition sequence. To reduce mismatch priming, specific adaptors and or primers are used to compensate for the degenerate restriction sites that may cause mismatch priming.

Nichols *et al*., Nature, (1994), vol. 369, no. 6480, pp. 492-493 discloses primers containing alternative nucleosides for use in PCR based methods. The article suggests the use of alternative nucleosides, in particular 3-nitropyrrole analogues to solve primer design problems that arise due to the degeneracy of the genetic code. The primers disclosed in the article allow for mismatch base pairing and the authors teach that the incorporation of alternative nucleosides, such as 3-nitropyrrole analogues in primers for PCR increases mismatch priming.

Object of the invention is to improve AFLP by reducing the occurrence of mismatch priming and/or reducing the number of bands which are obtained as a result of thereof, in particular when primers with selective bases, more specifically 2-10, in particular 4-8 selective bases are used.

According to the invention, it has now been found that this can be achieved by using AFLP-primers, which contain one or more "alternative nucleosides" or "nucleoside analogs" as further described hereinbelow. In general, these alternative nucleosides are analogues of the known naturally nucleosides such as adenine (A), thymidine (T), cytosine (C), guanidine (G), uracil (U), that bind either better or worse to the complementary base on the template, and/or bind less or non- selectively to more than one complementary base on the template strand.

Surprisingly, it has been found that even the use of alternative nucleosides which bind less strongly to the complementary base than the analogous nucleoside, and therefore destabilise the primer/template-duplex, can also improve selectivity, despite the fact that the use of such a less strongly binding nucleoside could by itself lead to a (deliberately induced) mismatch when it occurs at a selective base. Similar considerations hold for the use of non-selective nucleosides, which would also be expected to reduce selectivity.

In a first aspect the present invention therefore relates to the use of a selective primer containing 2-8 selective nucleotides at its 3' end, which primer further contains one or more alternative nucleosides, which is a nucleoside which has an alteration in the base nucleus, the sugar residue, the bond between the base nucleus and the sugar residue, and/or the linkage of the nucleoside to other nucleosides in the primer sequence or any combination thereof, compared to the naturally occurring nucleosides A, T, C and G and/or U, wherein the alternative nucleoside is located in a position 1-10 bases from the 3'-end of the primer sequence, in selective restriction fragment amplification (AFLP). More specifically, the one or more alternative nucleosides) will be 2-8 bases from the 3'-end of the primer, more preferably 4-7 bases from the 3'-end of the primer.

Although in principle most mismatch bands appear to be a result from mispriming on only one position, more than one alternative nucleoside can be incorporated in a primer of the invention. However, the primer will preferably contain less than three, more preferably less than two, even more preferably just one alternative nucleoside. If there are two or more alternative nucleosides present, they can either be adjacent to each other in the sequence of the primer, or separated by one or more conventional nucleotides/nucleosides. In this respect, it should be noted that - in a given situation- there may not be a close correlation between the number of mismatches and the number of alternative nucleosides to be incorporated in the primer according to the invention. By using an increased number of alternative nucleosides, their overall effect may increase and even a synergistic effect may be obtained.

The primer is preferably a selective primer, more specifically selective primer containing 2-10, preferably 4-8 selective nucleotides. The nucleoside analog (which can generally be in a position 1-10, preferably 2-8, more preferably 4-7 bases from the 3'-end of the primer sequence) can therefore be in the constant region or in the variable region of the primer sequence (depending upon the number of selective bases present).

Therefore, in general, whether the selective base is in the variable region or the selective region will depend upon the length of the variable region. For instance, in a +1 to +4 primer, the alternative base can be incorporated in the constant region of the primer, whereas in primers with 8 or more selective bases, it will generally be in the variable region.

However, it should be noted that according to the invention, the presence of an alternative nucleoside in the constant region can also improve selectivity, in particular when the nucleoside analog(s) is/are present in the first four bases adjacent to, more particular the two bases adjacent to the variable region ( if possible in view of both the possible positions for the nucleoside analog given above as well as the total number of selective bases present in the primer).

When a so-called non-selective nucleoside -as further described below- is used in the variable part of a primer, it is most preferably used in a position that has already been fixated by means of a pre-amplification.

The term nucleoside is used herein in its normal meaning in the art, i.e. a purine or pyrimidine base or analog thereof covalently bonded to a sugar residue or analog thereof, which may further contain a phosphite or phosphine group; and includes ribonucleosides, deoxyribonuclosides (which are preferred), and other nucleosides known per se.

The term nucleotide is also used herein in its normal meaning as a nucleoside containing a phosphate group or analog thereof. When in this disclosure mention is made of a nucleoside, it should be understood also the corresponding nucleotide can be used analogously in a manner known per se.

The alternative nucleosides as used herein are nucleosides which can have an alteration in the base nucleus (i.e. the purine or pyrimidine residue), the sugar residue, the bond between the base and the sugar, and/or the linkage of the nucleoside to other nucleosides in the primer sequence, or any combination thereof, compared to the naturally occuring nucleosides A, T, C, G and/or U.
This alteration should lead to altered binding affinity (i.e. stronger, weaker and/or less selective) of the primer or a part thereof to the corresponding sequence on the template, compared to the corresponding native primer.

Generally, because of its modified structure, the alternative nucleoside will itself have an altered binding capacity to the nucleoside in the corresponding position on the template, which leads to an alteration in the binding affinity of the primer as a whole. However, it is also possible that the presence of an alternative nucleoside in the primer (also or even predominantly) alters the binding capacity of any of the other nucleosides A, T, C and G in the primer sequence, in particular when these bases lie close to the alternative nucleoside in the primer sequence.

Furthermore, although the nucleoside analog will generally be able to bind to the corresponding base in the template sequence, i.e. by hydrogen bonding ( for instance through Watson-Crick base pairing or similar interactions), this is not required. For instance, the nucleoside analog can also comprise a spacer molecule or a similar structure which has virtually no binding capacity to a nucleoside on the template.

Also, the alternative nucleoside can have an alteration in the way it is connected (i.e bonded) to the other nucleosides in the primer, which as such may not alter the binding capacity of the sugar/base-nuclei of the alternative nucleoside itself, but can still influence the binding capacity of the primer as a whole. Such alterations in the bonds between the nucleosides will generally lead to alterations in the backbone of the primer and the formation of so-called "oligonucleotide analogs" as further discussed below.

It should be noted that during the amplification, the primer -and therefore the alternative nucleoside(s) contained therein- will be incorporated in the extension product produced by the polymerase reaction along the template. The strand thus obtained should itself then be able to serve as a template for a further cycle of amplification, in order to enable exponential amplification of the original strands, and the alternative nucleosides are preferably chosen so as to make this possible, as will be clear to the skilled person.

It will generally be preferred that the alternative nucleoside enables full extension of the template. This is because -even though AFLP is not predominantly directed to the detection of RFLP, however predominantly to the detection of point mutations either in the restriction site or in the selective nucleoside- every AFLP marker is predominantly identified by its length, so that full elongation is important. In such a case the alternative nucleotides present in the extension product will usually be at or near the 5'-end of the new template strand (as the primer used will form the 5'-end of the extension product).

What is generally observed when nitropyrrole-based primers are used is that only partly extension did not occur and a new product of predictable length was obtained. This product was of course not observed when the strands harbouring the alternative primer were visualized. Therefore, this does not affect too much the usefulness of the alternative primers based on the nitropyrrole nucleus.

However, any alternative nucleosides which could possibly interfere with the full extension of the primer should preferably still enable (at least some degree of) exponential amplification, i.e. the strands formed by primer extension should still be able to function to (at least some extend) as a template in a subsequent amplification cycle.

In general, the alterations which lead to the alternative nucleoside are generally the result of changes in the chemical structure of the purine or pyrimine base, the sugar residu, the covalent bond between the base and the sugar, and/or the linkage of the nucleoside with other nucleosides, compared to the naturally occuring nucleosides A, T, G, C and U.

These chemical alterations can comprise adding one or more atoms to the nucleoside structure, deleting one or more atoms from the nucleoside struture, and/or replacing one or more atoms from the nucleoside structure with one or more different atoms or bonds, adding one or more substituents to a nucleoside, changing the pattern of substitution or bonding of a nucleoside or the structural units thereof (base, sugar, linkage), as well as further known strategies for making nucleoside analogs, as well as any combination thereof. In particular, each of the structural units of the nucleoside can be replaced by a group analogous thereto, such as those known per se in the art of nucleotide chemistry.

For instance, the purine or pyrimidine nuclei can be replaced by the corresponding aza- and deaza-purine residues or aza- and deaza-pyrimidine residues, respectively; xantine and hypoxantine nuclei; inosine and nebularine nuclei; as well as other substituted or unsubstituted heterocyclic base analogs known per se, which are for instance discussed per se in WO 94/24114, WO 96/11937, WO 96/14329 and US-A-5,645,985, as well as the further references mentioned therein.

Also, the base nuclei can be substituted with any number of substituents known per se from substituted analogs of naturally occuring nucleosides, including substituents such as C₁-C₁₀-alkyl, C₁-C₁₀-alkenyl and C₁-C₁₀-alkynyl groups, halogen atoms, aromatic and heteroaromatic groups, nitro groups, thio groups, etc. These and other suitable substituents are discussed per se in for instance WO 94/24114, WO 96/11937, WO 96/14329 and US-A-5,645,985, as well as the further references mentioned therein.

For instance, the base nucleus can be replaced by a nitro-substituted -in particular 3-nitro substituted- nitropyrrole, diazole and triazole group, or another suitable nitro-substituted heterocyclic group, such as described in WO 94/06810 and US-A-5 438,131. Of this class of compounds, 3-nitropyrrole is particularly preferred.

Non-limiting examples of suitable base nuclei are the 7-deazapurine analogs, e.g. 7-deazaadenine, 7-deazaguanine, and 7-deazahypoxanthine; 9-deazapurine analogs, e.g. 9-deazaadenine, 9-deazaguanine, and 9-deazahypoxanthine; 8-azapurine analogs, e.g. 8-azaadenine, 8-azaguanine, and 8-azahypoxanthine, 5-azapyrimidines (1,3,5-triazines), e.g. 5-azacytosine, 5-azathymine, and 5-azauracil, 6-azapyrimidines (1,2,4-triazines), e.g. 6-azacytosine, 6-azathymine, and 6-azauracil; dideazaheterocyclic base analogs, e.g. 8-aza-7-deazapurines (pyrazolo-[3,4-d]pyrimidines), amino-imidazole carboxamide, appropriately derivatived 1,2,4-triazoles, thiazoles; selenazoles; methylated base analogs, e.g. 5-methylcytosine, 5-fluorouracil, 5-bromouracil, 5-chlorouracil, 5-iodouracil, 5-(carboxyhydroxylmethyl)uracil, 5-carboxymethylaminomethyl-2-thiouridine, 5-carboxymethylaminomethyluracil, methylpseudouracil, N2,N2-dimethylguanine, 2-methyladenine, 2-methylguanine, 4-N-methylcytosine, 5-methylcytosine, N6-methyladenine, 5-methylaminomethyluracil, 5-methoxyaminomethyl-2-thiouracil, 5'-methoxycarbonylmethyluracil, uracil-5-oxyacetic acid (v), pseudouracil, 2-thiocytosine, 2-thiouracil, 4-thiouracil, 5-methyluracil, and 2,6-diaminopurine, as well as 8-oxo-N⁶-methyladenine and 7-deazaxantine. These and other suitable base analogs are discussed per se in for instance WO 94/24114, WO 96/14329, WO 96/11937 and US-A-5,645,985 as well as the further references mentioned therein.

The sugar residu can be a 2'-deoxyribose moiety or any suitable analog thereof known per se from nucleoside chemistry. Such analogs include other pentose or hexose moieties, including D-ribose, 2'-O-alkyl, 2'-O-allyl, 2'-amino, 2'-halo functionalized sugar residues, as well as alternate structures such as a cyclopentane ring, a 6-member morpholino ring and the like. Sugars having a stereochemistry other than that of a D-ribose are also included.

Also, one or more of the hydroxyl groups in the sugar moiety can be replaced by -for example- a halogen, a heteroatom, an aliphatic groups, or are functionalized as ethers, amines, thiol and the like. Also, the sugar can carry groups such as the substituents mentioned above for the base nucleus, as well as other substituents for sugar residues known per se from nucleoside chemistry.

Also, the sugar moiety of a nucleoside can be replaced by a suitable carbocyclic group, or by non-cyclic carbohydrate groups known per se as analogs of sugar residues in nucleoside chemistry.

The above and other modifications to the sugar residu are discussed per se in for instance WO 94/24114, WO 96/14329, WO 96/11937 and US-A-5,645,985 as well as the further references mentioned therein.

In particular, the sugar moiety can be replaced by a corresponding ring-opened sugar residu, such as a alicyclic pentose (including alicyclic ribose) and hexose residues.

The bonds between the nucleosides in the primer can be replaced by any analog of the native phosphodiester group that can covalently couple adjacent nucleomonomers, including such "substitute linkages" as phosphodiester analogs, e.g. phosphorothioate, methylphosphonate and phosphoramidate linkages; as well as nonphosphorus containing linkages, e.g. such as acetals and amides. The nucleosides can also be bonded via a 2'-5' linkage instead of the normal 3'-5' linkage. These and other alterations in the way the nucleotides in the primer are linked to each other, which will generally lead to alterations in the primer backbone, are discussed per se in for instance WO 94/24114, WO 96/11937, WO 96/14329, and US-A-5,645,985, as well as the further references mentioned therein.

The constituents of the "backbone" of the primer can also be modified or even replaced by one or more amino acids, peptides or peptide groups and/or peptide bonds so as to provide so-called "peptide nucleic acids" ( PNA), as for instance described by B. Hyrup and P.E. Nielsen (Bioorganic and Medicinal Chemistry (1996), vol.4, no. 1, pp.5-23) and by J.P. Finn et al (Nucleic Acids Research, vol.24, no.17, 3357-3363 (1996). These PNA's may be based upon the native nucleosides C, G, T or A, or may further contain analogs thereof, such as the nuclei disclosed herein.

The alternative nucleosides used according to the invention can also contain removable protective groups known per se for nucleosides and nucleoside analogs, including the enzymatically removable groups of EP-A-0 649 855.

From the above, it will be clear that the invention is not particularly limited to the alternative nucleoside(s) used: in principle, all modified nucleosides that can suitably be incorporated into a primer and that alter the binding affinity of said primer to the template can be used, as long as they lead to a reduction of mismatch priming or mismatch bands. The invention also comprises a method and assay for identifying promising candidates by comparing fingerprints generated by +n and +(n+1) fingerprints, as further discussed below.

The alternative nucleosides used according to the invention are commercially available and/or can be prepared in a manner known per se from nucleoside chemistry, or analogous methods. For instance, they can be prepared de novo from suitable starting materials, and/or by by (further) derivatisation of native nucleosides A, T, C, G, U, or known synthetic nucleosides. A number of such nucleoside analogs have been described in the art for use in PCR primers. According to the invention, they are now used to reduce mismatch priming in amplification techniques, in particular in AFLP, for which purpose they have not been suggested before.

Examples of suitable alternative nucleosides are given in for instance WO 94/24114, WO 96/11937, WO 96/14329 and US-A-5,645,985 as well as the further references mentioned therein.

In general, the alternative nucleosides used in the invention can be distinguished as selective and non-selective nucleosides. Selective nucleotides are nucleotides which are capable of binding to, or preferably bind to, only one of the bases A, T, C, G present on the template. Non-selective nucleotides are capable of binding more than one or even all of the bases A, T, C, G on the template strand.

Typical examples of aselective bases include the so-called "universal nucleotides" known in the art, such as 1,2-dideoxyribofuranose, 1,2-dideocy-1(C)-phenylribofuranose, the deoxyribose of benzimidazole, hypoxantine, xantine, guanine deoxyribonucleosides, 5-fluorodeoxyuridine (pairing with A and G only), (deoxy)inosine, etc. Other suitable universal nucleosides include the various nitropyrrole-, nitrodiazole-, nitrotriazole- and other nitro-substituted heterocyclic group containing nucleosides described in WO 94/06810 and US-A-5 438,131.

The alternative nucleosides used in the invention can further be classified as stabilizing and destabilizing nucleosides. Stabilizing nucleosides will as a rule bind more strongly to the complementary base on the template than the native A, T, C or G, and/or through their presence stabilize the template/primer duplex. Destabilizing nucleosides will bind less strongly to the complementary base, and/or generally provide for destabilization of the primer/template-hybrid.

The influence that the presence of a particular alternative nucleoside in a primer has on the binding strength can be determined in a manner known per se. For instance, the melting temperature (Tₘ) of a template/primer duplex, in which the primer contains the alternative nucleoside, can be compared to the Tₘ for the template/primer-duplex, in which the primer contains the nucleotide A, T, G or C naturally complementary to the nucleotide present on the template. A lower Tₘ for the primer analog will mean reduced binding strength compared to the native nucleoside, a higher Tₘ a higher binding strength.

Examples of selective, stabilising alternative nucleosides useful in the invention are: nucleosides containing a 2,6-diaminopurine nucleus, such as 2,6-diaminopurine, 5-Methyl-2'-deoxyCytidine and 5-propynyl-2'-deoxyCytidine.

Examples of selective, destabilising alternative nucleosides useful in to the invention are: N6-Methyl-2'-deoxyAdenosine, 7-deaza-2'-deoxyAdenosine, 2-OMethyl-deoxy-AdenosinedA, 8-Bromo-2'-deoxyAdenosine 8-Bromo-2'-deoxyGuanosine, 5-Bromo-2'-deoxyCytidine, 5-iodo-2'-deoxyCytidine, 5-Bromo-2'-deoxyUridine, 5-iodo-2'-deoxyUridine, 5-Fluoro-2'-deoxyUridine O6-Me-2'-deoxyGuanosine, O4-Me-Thymidine, 7-deaza-2'-deoxyguanosine, 3'-deoxyAdenosine, 8-oxo-2'-deoxyAdenosine, 7-Deaza-2'-deoxyGuanosine 8-oxo-2'-deoxyGuanosine, 2'-deoxyUridine, 2-Amino-2'-deoxyAdenosine, 4-thio-deoxythymidine, 2-thio-deoxythymidine, as well as 2-aminopurine nucleus containing nucleosides.

Examples of non-selective, destabilising alternative nucleosides useful in the invention are: dSpacer, 2'-deoxynebularine, 2'-deoxyInosine, as well as the universal nucleotides described above, including those with 3-nitropyrrole, 4-methylindole or 5-nitroindole groups replacing the base nucleus.

Preferred alternative nucleosides are 2,6-diaminopurine, N6-Methyl-2'-deoxyAdenosine, 7-deaza-2'-deoxyAdenosine, 2-OMethyl--2'-deoxyAdenosine, 8-Bromo-deoxyAdenosine, spacer groups, and 5-nitroindole containing nucleosides, as well as nucleosides based on inosine or the inosine nucleus. Another particularly preferred class are those which contain 3-nitropyrrole-, 3-nitrodiazole-, 3-nitrotriazole- and other 3-nitro-substituted heterocyclic groups as the base nucleus analog; 3-nitropyrrole being especially preferred. Alternative nucleosides based upon the difluorotoluene nucleus may also be of particular value.

Currently, the best results are obtained using inosine or other alternative nucleosides based upon the inosine nucleus.

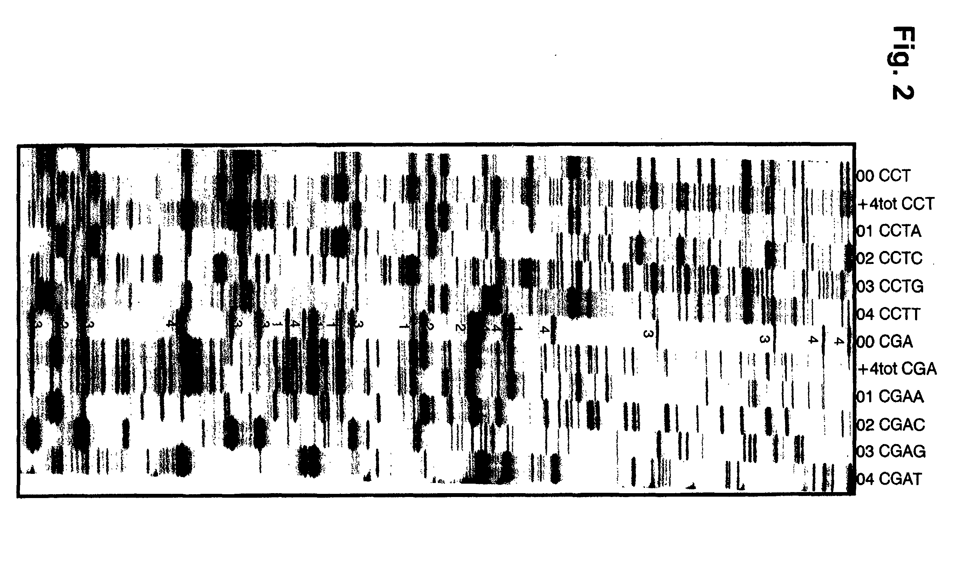

A further particularly preferred class of alternative nucleosides are those in which the sugar moiety has been replaced by a corresponding ring-opened sugar residu, such as an alicyclic pentose or hexose residu. As the base nucleus, these alicyclic nucleosides can contain either the native bases A, T, G and C (which are preferred) , as well as U and all other analog base nuclei described above. The structure and synthesis of such alicyclic nucleosides are described in Herdewijn et al, J. Med. Chem., 35, 1458 (1992) and Koster et al. Nucl. Acids Res. 12, 4539 (1984), and an exemplary synthesis is shown in figure 2. Particularly preferred are such alternative nucleosides containing an alicyclic ribose as the sugar moiety, and A, T, C or G as the base.

Another particularly preferred class of alternative nucleosides may be the peptide nucleic acids described hereinabove.

Of course, several of the abovementioned modifications or alterations to the native nucleoside/nucleotide structure may also be combined to provide further valuable alternative nucleosides of the invention. For instance, the preferred base nucleus analogs, such as nuclei of the inosine or nitropyrrole type, may be combined with ring opened sugar residues as described above.

In a specific embodiment of the invention, the alternative nucleosides are such that they can also serve as a detectable marker for the nucleic acid strand in which they are incorporated, using any suitable physical, chemical or biological detection technique known per se.

For instance, the alternative nucleosides can contain one or more radio-active atoms so as to provide for radio-labelling, in which case the alternative nuceleosides may be analogs of known nucleosides that are easier to provide synthetically with a radio-active atom incorporated in their structure at the desired position than the corresponding native nucleoside A, T, C or G.

The alternative nucleosides can also be such that they can be detected and/or distinguished based upon their physical and/or chemical structure, for instance because of their chemical properties (i.e. susceptibility to specific chemical reactions or reagents or specific enzymes used in detection), their physical properties (i.e. so as to provide -for instance- a highly specific pattern of nucleic magnetic resonance) or the presence of specific detectable groups or structures, such as fluorescent of phosphorent groups, or groups or structures which can absorb radiation of a specific frequency so as to enable spectroscopic detection. Another way in which the alternative nucleoside can serve as a detectable marker is that it provides for a distinguisable and/or detectable extension of the primer, for instance by acting as a stop for the polymerase, resulting in "shortened" extension products.

It will be clear that when such a detectable alternative nucleoside is used, the detection of the amplified fragments can also take place on the basis of the specific properties of said marker nucleoside as incorporated in the amplified fragments. Therefore, according to this embodiment, detection of the amplified fragments need not take place by gel-electroforesis or a similar technique. In this aspect, the invention relates to a method for the specific amplification of PCR fragments, in particular AFLP fragments, by means of amplification, in particular AFLP, in which a primer is used that contains one or more detectable alternative nucleosides as disclosed herein, and in which the detection and/or identification of the amplified fragments takes place based upon the specific properties of the detectable alternative nucleosides.

In this specific embodiment, any alternative nucleoside as disclosed herein that makes it possible to distinguish between the nucleic acid strand(s) in which it has been incorporated (i.e. during amplification) and nucleic acid strand(s) in which it is not present (i.e. the fragments of the original mixture) using a suitable detection technique, can be used as a marker.

The primers containing the alternative nucleosides can be prepared in a manner known per se, for instance by incorporating the alternative nucleoside in the primer sequence during the synthesis thereof. Conventional techniques for preparing oligonucleotides can be used, including automated chemical synthesis based upon phosphoamidite or phosphotriester chemistry; solid phase methods; as well as techniques such as nick translation, random priming or preparation of oligonucleotides via PCR. These and other suitable techniques for the synthesis of oligonucleotides are discussed per se in for instance WO 94/24114, WO 96/11937, WO 96/14329 and US-A-5,645,985, as well as the further references mentioned therein.

It is also possible to derivatize a standard AFLP primer, so as to convert one or more of the nucleosides A, T, G or C present therein into an alternative nucleoside according to the invention, for instance via base-specific or site-specific derivatisation, either chemically or enzymatically. For instance, chemical or enzymatical methylation can be used.

Usually, the alternative nucleoside will be used to replace, in a known primer sequence, one or more of the nucleotides A, T, C or G.

Preferably, the native nucleotide A, T, G or C will be replaced by either a known selective analog thereof, or by a non-selective nucleoside. For instance, each of A, T, C or G in a known primer sequence can be replaced by a corresponding aza or de-aza analog, a substituted analog or a alicyclic ribose containing analog as described above, as well as by a non-selective 3-nitropyrrole containing nucleoside. However, as will be clear from the above, this is not required, and non-analogous and/or destabilising nucleosides as well as spacer molecules can also be used, both in the constant as well as in the variable region.

In general, when a selective base in a known primer is replaced by a known selective analog of A, T, C or G, the selectivity of the primer will be maintained or even improved. Nevertheless, according to the invention, even when a selective base is replaced by a destabilising nucleoside or a non-selective nucleoside, a reduction in mismatch priming and/or mismatch bands can also be obtained. However, there may also be a fixed sequence after AFLP preamplification allowing for non-selective alternative nucleosides in the variable part of the primer; otherwise, unwanted amplification could possibly be induced.

In practice, it has been found that using destabilising alternative nucleosides such as inosine- based nucleosides may even provide results superior to stabilising alternative nucleosides.

Also, when a known AFLP-protocol is to be improved by reducing the occurrance or intensity of mismatch bands, it will generally be preferred to replace one or more nucleosides in the known primer sequence with a known selective analog thereof, in order to assure amplification of the same fragments as amplified with the known protocol.

Nevertheless, it should be remembered that in general, selective bases in AFLP primers can be chosen at random or by means of trial and error, and the same holds for alternative nucleosides used in the variable region in the primers of the invention.

In general, the alternative nucleoside containing primers of the invention can be considered "oligonucleotide analogs" as defined in for instance the international applications WO 94/22894 and WO 94/22883, i.e. as compounds which function like oligonucleotides but have non-naturally occurring portions, i.e. altered base moieties, sugar moieties and/or altered inter-sugar linkages.
Accordingly, the primers of the invention, which are oligonucleotides falling within this general definition, can be considered "primer analogs".

These analog primers can be used in AFLP in a manner known per se for AFLP-primers. In particular, they can be used in conjuction with the adapters for which the corresponding "native" primer is usually used, or otherwise to replace the native primer in a known protocol.

The AFLP-amplification itself is further carried out in a manner known per se, for instance as described in EP-A-0 534 858, and the resulting amplified fragments can be visualised. The resulting fingerprint should show less mismatch bands, and/or a reduction in the intensity of any mismatch bands, compared to fingerprints obtained using the corresponding native primer.

As is known per se in AFLP-technology, the use of some primers will provide more informative fingerprints compared to others, depending upon factors such as the starting DNA used (e.g. its origin), the restriction enzyme(s) used, the adapters used, the primers used, and in particular the specific selective bases used and the number thereof. Also, the occurrance of mismatch priming events using "native" primers may vary, again depending upon the above factors, as well as further factors known per se that can influence the stability of primer/template duplexes, such as the relative or absolute amount of CG-pairings in the duplex, the hybridisation conditions used, length of the primers, the possible formation of secondary structures in the template strand etc..

Similarly, it is possible that some primers of the invention containing alternative nucleosides will give better results than others, i.e. lead to more informative fingerprints and/or give rise to less mismatch priming events. This will not only depend upon the aforementioned factors, but particularly on the specific analog primer used, i.e. the number, nature and position of the alternative nucleosides present therein, possibly in conjunction with the hybridising conditions used.

As will be clear, it is even possible that some analog primers of the invention can give, in a specific application, worse results than the corresponding native primer. However, a skilled person will be capable -on the basis of the disclosure herein- to select analog primers of the invention best suited for each specific application, without diverging from the scope and the general inventive concept of the present invention.

In general, the specific analog primers to be used for any given amplification can be selected by the skilled person, for instance starting from the known primers, and based upon general experience, the properties of the analog nucleosides used as well as the disclosure herein. The selection of the analog primers may also involve routine experimentation or even a certain amount of trial and error, i.e. by repeating a given AFLP-reaction with one or more analog primers of the invention until a more informative fingerprint and/or reduction of mismatch bands is obtained. For instance, analog primers with more (or less) alternative nucleosides, with different alternative nucleoside(s), with the alternative nucleoside(s) in different positions in the primer sequence, and/or with other alterations in the analog primer sequence (i.e. its total length, the number selective bases and the specific selective bases used) can be tried.

Also, the present application provides specific criteria as well as a specific assay for determining and/or predicting the influence that the presence of an alternative nucleoside, as well as its position in the primer sequence, will have on the occurrence of mismatch priming.

In this assay, the number of mismatch events (bands) obtained with a primer analog of the invention is compared to the number of mismatch events (bands) obtained with the corresponding native primer.

In doing so, the occurrance of mismatch events (bands) can be determined -for the primer analog and the native primer seperately- by carrying out a set of a single +n and four +(n+1) amplifications (for instance one +3 and four +4-amplifications). In this set of five amplifications -which are preferably run in parallel- four +(n+1)-primers corresponding to the +n-primer are used, each having (essentially) the same sequence as the +n primer but having one of the native nucleosides A, T, C or G, respectively, at its 3'-end as a further selective base, i.e. +n-A, +n-T, +n-C and +n-G (collectively indicated as +n-N primers).

Thus, five fingerprints are obtained (preferably in parallel lanes of the same gel) which are then compared. Due to the presence of a further selective base, the four +n-N primers will provide less bands than the +n-primer; however, the fingerprints obtained with each of the four +n-N primers should combined give exactly the same (number of) bands as the +n fingerprint. Any further bands will be due to mismatch events, thus providing a quantative measure of the mismatch events in the AFLP-reaction used.

In the assay of the invention (at least) two such sets of five +n/+(n+1) fingerprints are run. In the first set of five, a primer analog of the invention is used as the +n-primer, together with the four corresponding +n-N analog primers. In the second set of five, the equivalent native +n and four +n-N primers are used. The number of mismatch bands obtained with the primer analog is than compared to the number of mismatch bands obtained with the native primer under the same circumstances. In this way, it can be determined whether the use of an analog primer of the invention -i.e. the presence of one or more alternative nucleosides therein and/or the position thereof- reduces the number of mismatch events.

This assay, which is further described in the Experimental part, can be used to generally identify promising compounds for use as alternative nucleosides in AFLP-primers and/or to generally identify positions in AFLP-primers that are susceptible to replacement by an alternative nucleoside or by the specific alternative nucleoside(s) used. For such general information, results from several assay's can be combined.

In a more directly applicable aspect, the assay can be used to obtain such information for a specific primer, a specific application and/or a specific AFLP-reaction. In any case, the assay of the invention will identify primer analogs that provide a reduction of mismatch events under the conditions of the assay (i.e. starting DNA, restriction fragments generated therefrom, adapters used, etc.). As these conditions usually (are chosen so as to) correspond to the intended final AFLP-reaction, the analog primers thus identified can immediately be put to this final use.

In a further aspect the invention relates to a method for carrying out AFLP, in which an analog primer according to the invention is used to replace a known AFLP primer. Preferably, besides the presence of the one or more alternative nucleosides as defined hereinabove, the alternative primer and the native primer will have identical sequences.

A further aspect of the invention lies in the fingerprint generated by the method described above, and/or by using the analog primers described herein, as well as any photographic print or other representation thereof.

In a further aspect, the invention relates to specific amplification of PCR fragments, in particular AFLP fragments, that are not detected through gel electroforesis as a method to generate a fingerprint, but by any other means. This aspect of the invention can comprise the use of a labelled or otherwise detectable alternative nucleoside as disclosed hereinabove.

In another aspect the invention relates to kits for AFLP, comprising at least one analog primer as described here-inabove, in combination with the adapters with which the analog primers of the invention are intended to be used (i.e. with which the constant region of the analog primer can hybridize). The kits can further contain all known components for AFLP kits, such as restriction enzymes (in which case the adapters are preferably suited to be ligated to the restrictes sites generated with said enzyme); a polymerase for amplification, such as *Taq*-polymerase; nucleotides for use in primer extension; as well as buffers and other solutions and reagentia; etc.. Further reference is made to the European patent application 0 534 858.

The invention will now be further illustrated by means of the Experimental part and the Examples contained therein, as well as the figures, in which:
- Figure 1 is a schematic representation of template/primer mismatch and mismatch priming;
- Figures 2-5 show DNA-fingerprints obtained from AFLP-reactions
- Figure 6 shows an exemplary synthesis of the alicyclic nucleoside analogs useful in the invention;
- Figure 7 again shows a DNA-fingerprint obtained from an AFLP-reaction.

Finally, although the present invention will be described hereinbelow with respect to AFLP, it is contemplated that the teaching of the invention can be applied to any priming event, in which the occurrance of mismatch priming is to be avoided or reduced. The invention can therefore be of use in any technique in which, following the formation of a template/primer hybrid, extension of the primer along the template is to be carried out, including all amplification techniques known per se.

### Experimental part

### A. Introduction

Mismatch rarely occurs in standard AFLP-reactions directed to obtaining about 50-100 AFLP-fragments (bands) in an AFLP-fingerprint following amplification. In most AFLP-reactions, in particular in plants, this is realized when AFLP primers are used with an extension of 3 selective nucleosides (so-called "+3-primers"). However, when primers with more than three selective nucleosides are used on the same preamplified template, sometimes the formation of mismatch bands can become a problem, which can lead to "contaminated" fingerprints and/or loss of selectivity. This becomes clear when AFLP fingerprints obtained using +3-primers are compared to AFLP fingerprints obtained using +4-primers, which contain the same three selective bases as the +3-primer, and furthermore A, C, G, or T, respectively, as the fourth selective nucleoside. When these fingerprints of the four separate AFLP-amplifications using these four +4-primers are compared to the +3-fingerprint, only bands from the original +3-fingerprint should be present in the +4-fingerprint. In other words, the four +4-fingerprints should together give the original +3-fingerprint. However, due to AFLP mismatch, there are contaminating bands in the fingerprints of the +4 primers as can be seen from the fingerprint shown in figure 2. Unexpectedly, this problem appears to increase when the number of selective bases in the primer is increased. When more than three, in particular four or more selective nucleosides are used, the generation of mismatch bands can compromise reliability.

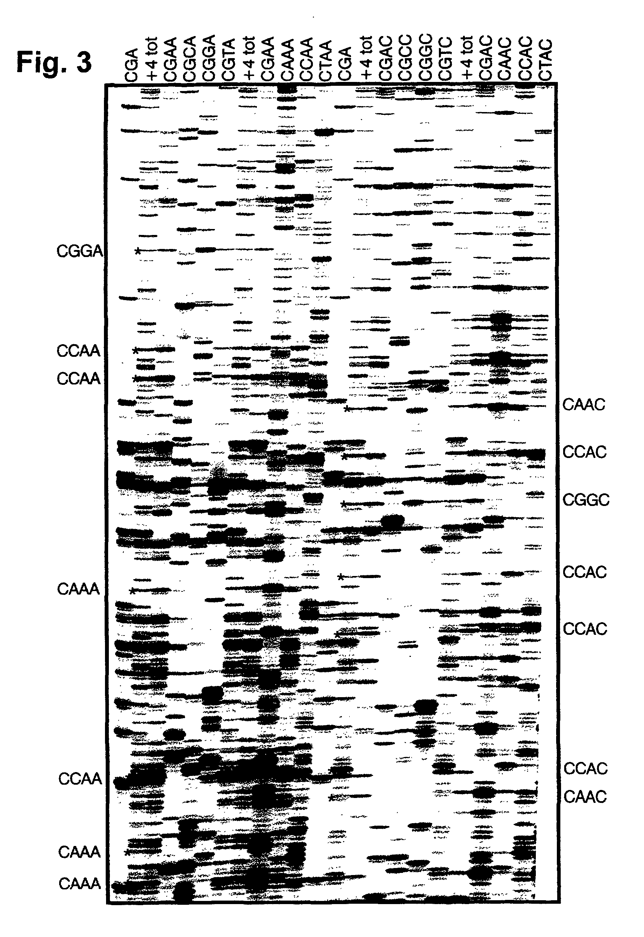

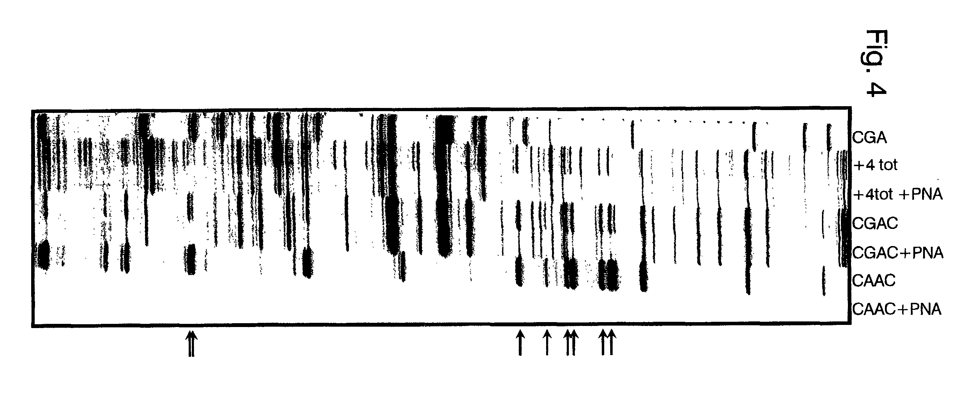

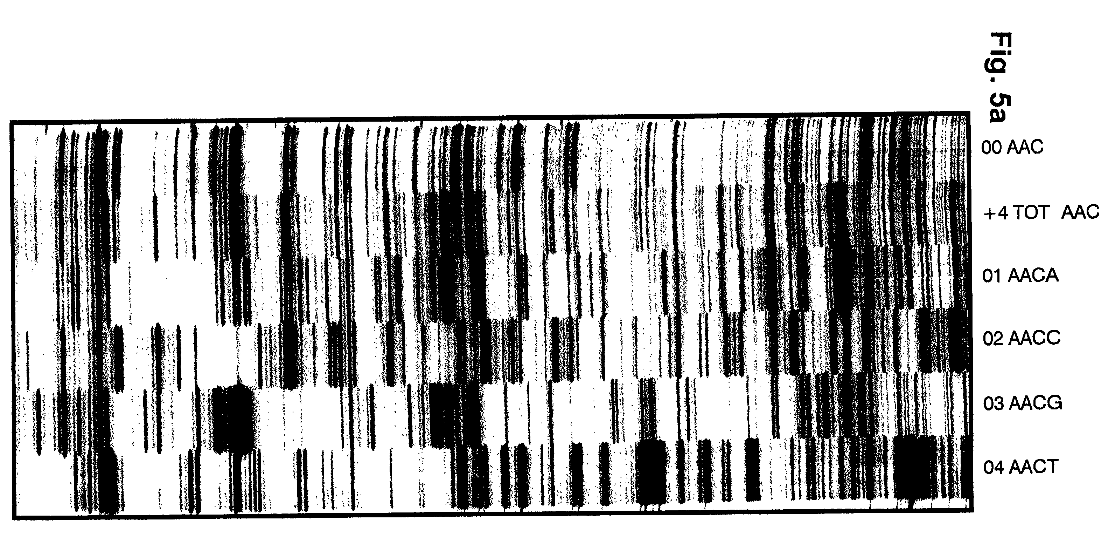

In principle, most mismatch bands appear to be a result from mispriming on only one position (Fig. 3, 4). Also, there appears to be some difference in frequency of mispriming on identified template sequences: this could be related to the structure of the template sequence, to sequence abundance or sequence preference.

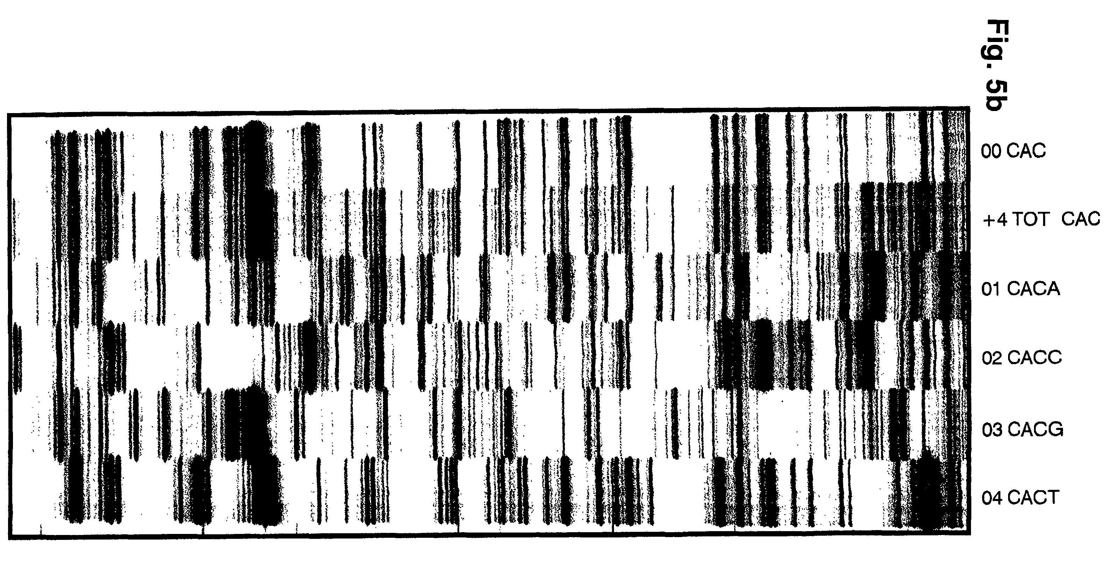

Also, it appears that mispriming is not dependent upon the sequence which precedes the selective nucleosides. This was found using templates prepared with other restriction enzymes for amplification using +4-primers (Fig. 5).

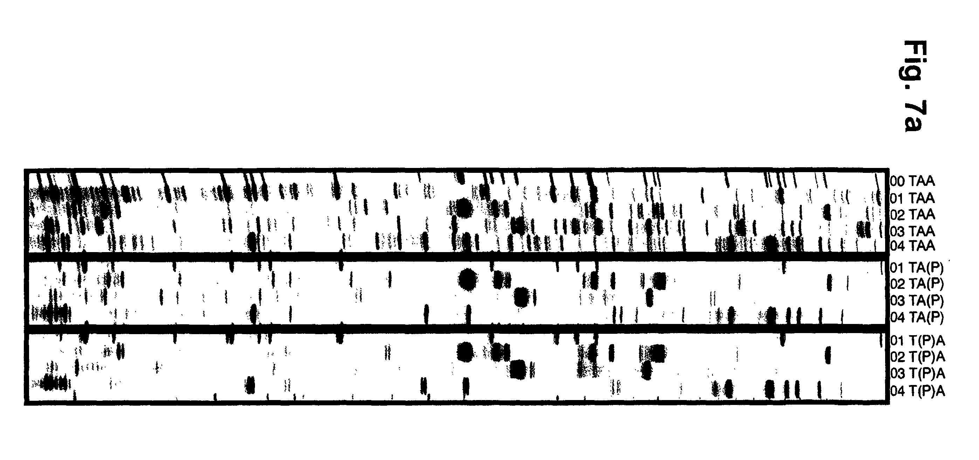

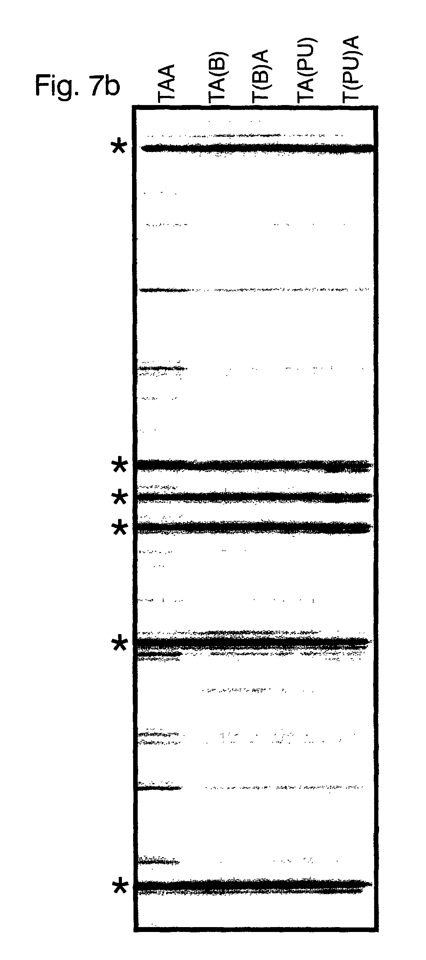

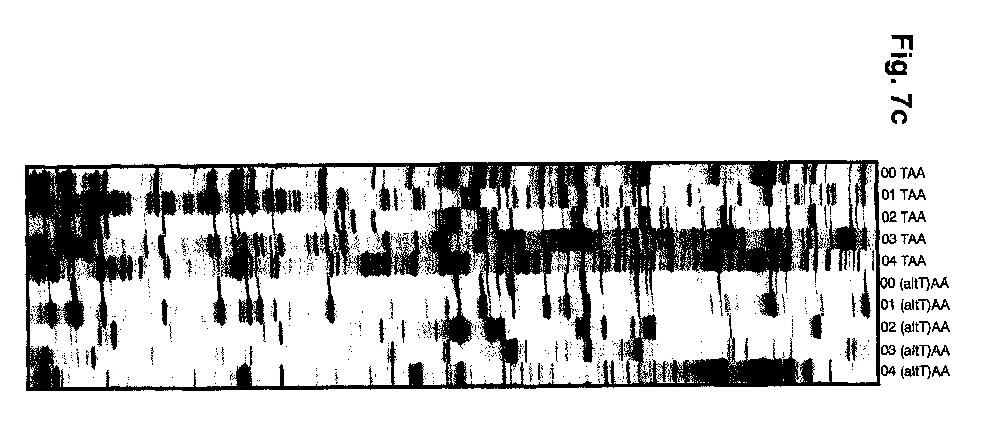

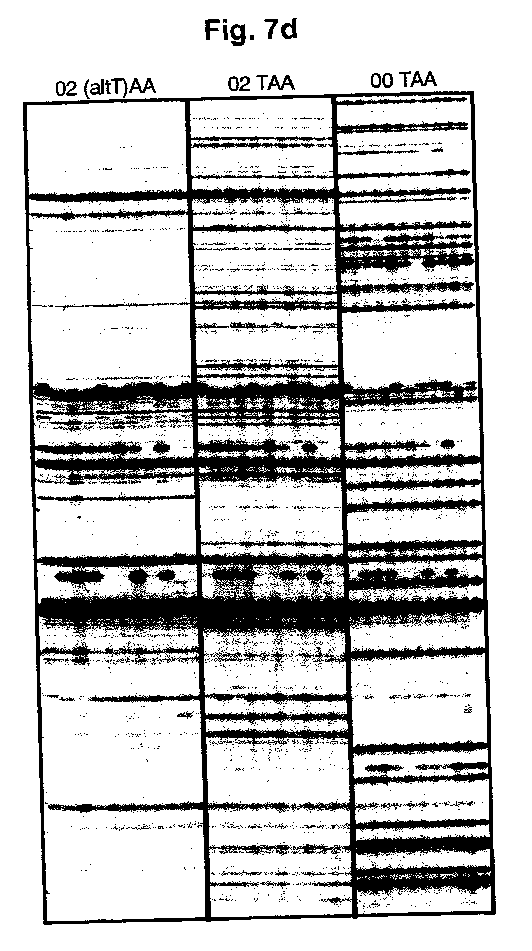

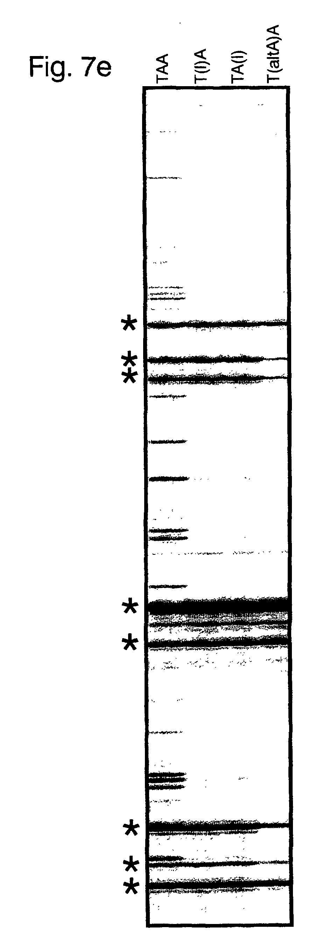

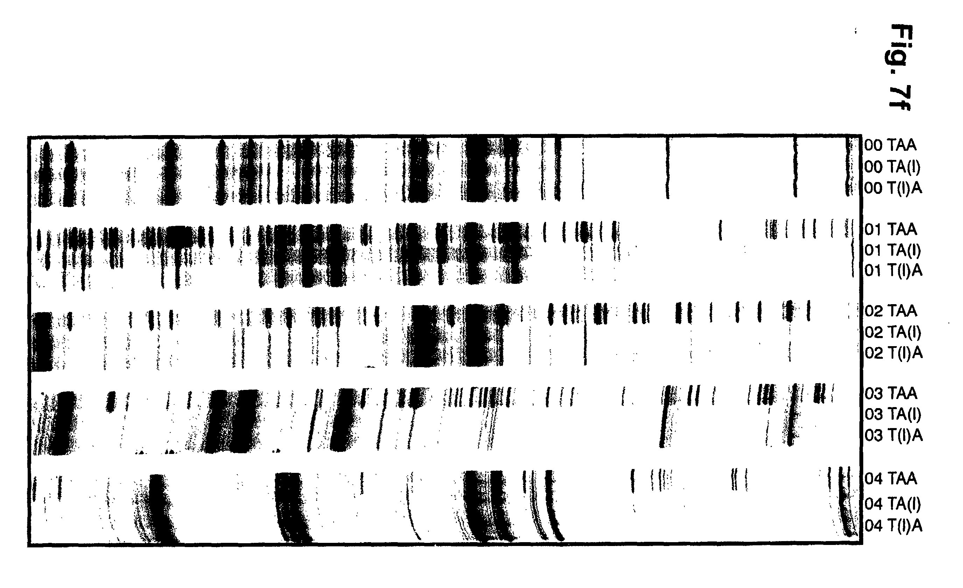

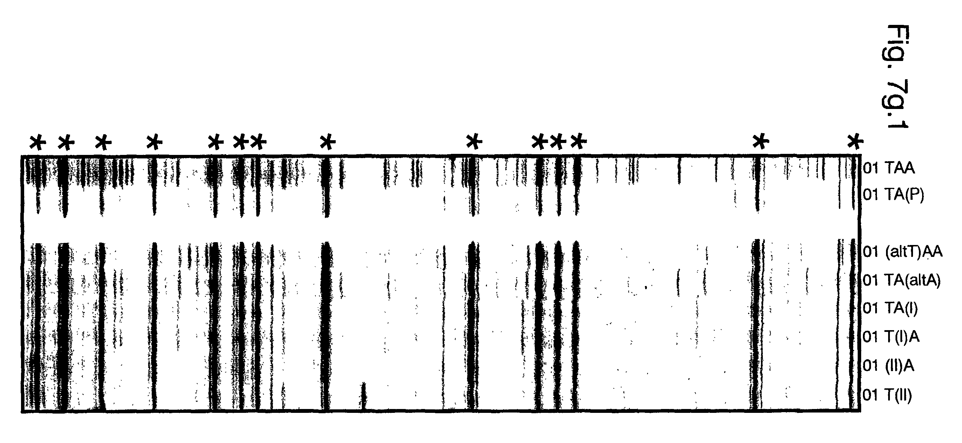

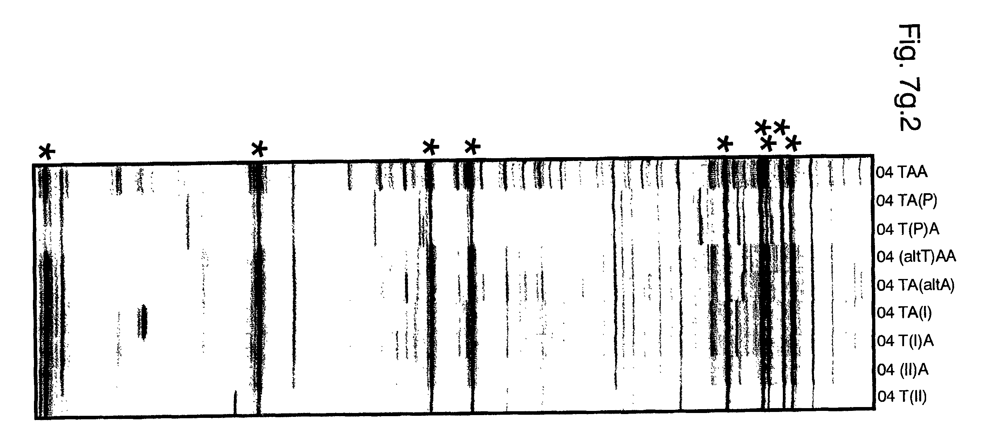

Suitable primers can be selected by means of the following criteria:
- Using alternative primers, it should be possible to obtain fingerprints, i.e. the alternative nucleoside should be recognized as a nucleoside by the thermostable polymerase used in the PCR reaction.
- The selectivity of the primers should be improved: i.e. less mismatch bands should be obtained. This can be analyzed using the assay described herein for +3 and +4 primers, i.e. that AFLP using alternative +4-primers generate less mispriming bands than the corresponding standard +4-primers (fig.7). The same applies to primers with more than 4 selective nucleosides: by increasing the number of selective nucleosides by one every time, the bands in the fingerprint must be a subset of bands in a fingerprint without the increased number of selective nucleosides and fingerprints with primers extended with either A, C, G or T, respectively, must together have only the bands present in the fingerprint without the increased number of selective nucleosides.

### B. Rationale behind the use of extended primers.

The rationale behind the use of extended primers inter alia lies in the possibility of multiplexing primers: often the maximum resolution on a polyacryl gel is reached using +3 AFLP primers. When the number of fragments is reduced by using extended primers more than two primers could be employed in a reaction. The number of primers employed in a reaction could increase 4ⁿ with the increase in the number of selective nucleosides (n). Increasing the number of informative fragments would largely improve the information content of a fingerprint. The rationale behind the use of extended primers also lies in achieving an increase in the concentration of certain fragments, with the aim of increasing the detection limit for these fragment. This becomes relevant in particular when different labels or different detection systems for generating a fingerprint are used than described herein, that might be less sensitive.
For some genomes, +3 primers can not be used since they generate too many fragments to resolve on a polyacrylgel (more than one hundred) . Increasing the number of selective nucleosides could solve this problem, if selectivity of primers is guaranteed.

### C. Detailed explanation of the Figures.

Figure 1 shows a schematic representation of template/primer match (on top) and mismatch priming at +1 (MM+1), +2 (MM+2), +3 (MM+3) and +4 (MM+4) positions in the primer.

Figure 2 shows AFLP -fingerprints after amplification with radioactively-labeled *Eco*RI-primer 00AAC and *Mse*I-primers 00CCT, +4totCCT, 01CCTA, 02CCTC, 03CCTG, 04CCTT, 00CGA, +4totCGA, 01CGAA, 02CGAC, 03CGAG, 04CGAT, respectively. To generate the fingerprints in the lanes +4totCCT and +4totCGA, primers , 01CCTA, 02CCTC, 03CCTG, 04CCTT and 01CGAA, 02CGAC, 03CGAG, 04CGAT, respectively, were pooled in equimolar amounts and the total concentration of *Mse*I-primer in those reactions was kept the same as when only one *Mse*I-primer was used. AFLP reactions were preceeded by a +1+1 preamplification. This picture illustrates that +4 primers do not generate a subset of the fragments generated by the corresponding +3 primer, however, many contaminating bands are present. Therefore the four +4 primers with A, C, T and G, as the fourth selective nucleoside do not together generate the corresponding +3 fingerprint, which is also clear from the fingerprints generated after amplification with +4totCCT and +4totCGA. For every band in the 00CGA fingerprint it is indicated what the fourth selective nucleoside on a *Mse*I +4 primer should be to generate that specific band: 1 indicates an A (the band is generated with primer 01CGAA), 2 indicates a C (the band is generated with primer 02CGAC), 3 indicates a G (the band is generated with primer 03CGAG), and 4 indicates a T (the band is generated with primer 04CGAT).

EcoRI primer 00AAC | 5'-GACTGCGTACCAATTC AAC-3'
MseI primer 00CCT | 5'-GATGAGTCCTGAGTAA CCT-3'
MseI primer 01CCTA | 5'-GATGAGTCCTGAGTAA CCTA-3'
MseI primer 02CCTC | 5'-GATGAGTCCTGAGTAA CCTC-3'
MseI primer 03CCTG | 5'-GATGAGTCCTGAGTAA CCTG-3'
MseI primer 04CCTT | 5'-GATGAGTCCTGAGTAA CCTT-3'
MseI primer 00CGA | 5'-GATGAGTCCTGAGTAA CGA-3'
MseI primer 01CGAA | 5'-GATGAGTCCTGAGTAA CGAA-3'
MseI primer 02CGAC | 5'-GATGAGTCCTGAGTAA CGAC-3'
MseI primer 03CGAG | 5'-GATGAGTCCTGAGTAA CGAG-3'
MseI primer 04CGAT | 5'-GATGAGTCCTGAGTAA CGAT-3'

Figure 3 shows DNA-fingerprints generated with radioactively-labeled *Eco*RI-primer 00AAC and *Mse*I-primers CGA, +4tot, CGAA, CGCA, CGGA, CGTA, +4tot, CGAA, CAAA, CCAA, CTAA, CGA, +4tot, CGAC, CGCC, CGGC, CGTC, +4tot, CGAC, CAAC, CCAC, CTAC, respectively, that are able to amplify similar bands, as indicated. Bold characters in the primer sequences indicate the difference relative to the CGAA and CGAC primer. To generate the fingerprints in the lanes +4tot, primers CGAA, CGAC, CGAG, and CGAT, were pooled in equimolar amounts and the concentration of *Mse*I-primer in those reactions was kept the same as when only one *Mse*I-primer was used. AFLP reactions were preceeded by a +1+1 preamplification. Bands indicated with an asterix are mismatch bands in the +4tot fingerprints: +4 sequences at the left and right side of the figure indicate the +4 extensions that would be extensions for match primers to generate those fragments as deduced from the fingerprints generated with the primers having these extensions as indicated on top of the figure.

By identifying most of the mismatch templates that could explain mismatch bands in fingerprints generated with +4tot, CGAA, CGAC, CGAG, and CGAT, it was concluded that mismatch mostly occurs at one position in template molecules.

EcoRI primer 00AAC 5′-GACTGCGTACCAATTC AAC-3′
MseI primer CGA: 5′-GATGAGTCCTGAGTAA CGA-3′
MseI primer CGAA: 5′-GATGAGTCCTGAGTAA CGAA-3′
MseI primer CGCA: 5′-GATGAGTCCTGAGTAA CGCA-3′
MseI primer CGGA: 5′-GATGAGTCCTGAGTAA CGGA-3′
MseI primer CGTA: 5′-GATGAGTCCTGAGTAA CGTA-3′
MseI primer CAAA: 5′-GATGAGTCCTGAGTAA CAAA-3′
MseI primer CCAA: 5′-GATGAGTCCTGAGTAA CCAA-3′
MseI primer CTAA: 5′-GATGAGTCCTGAGTAA CTAA-3′

MseI primer CGAC: 5′-GATGAGTCCTGAGTAA CGAC-3′
MseI primer CGCC: 5′-GATGAGTCCTGAGTAA CGCC-3′
MseI primer CGGC: 5′-GATGAGTCCTGAGTAA CGGC-3′
MseI primer CGTC: 5′-GATGAGTCCTGAGTAA CGTC-3′
MseI primer CAAC: 5′-GATGAGTCCTGAGTAA CAAC-3′
MseI primer CCAC: 5′-GATGAGTCCTGAGTAA CCAC-3′
MseI primer CTAC: 5′-GATGAGTCCTGAGTAA CTAC-3′

Figure 4 shows DNA-fingerprints generated with radioactively-labeled *Eco*RI-primer 00AAC and *Mse*I-primers CGA, +4 tot, CGAC, CAAC, respectively. Every two lanes starting with +4 show fingerprints generated with the same *Mse*I-primer, however minus and plus a PNA oligo added that specifically blocks template amplified with *Mse*I +4 primer CAAC. This figure shows that indeed part of the template amplified with primer CAAC is also amplified with primer CGAC and primers +4tot, since similar bands are blocked in all fingerprints as indicated with arrows. In the lanes +4tot, primers CGAA, CGAC, CGAG, and CGAT, were pooled in equimolar amounts and the total concentration of *Mse*I-primer in those reactions was kept the same as when only one *Mse*I-primer was used. AFLP reactions were preceeded by a +1+1 preamplification.

EcoRI primer 00AAC          5'-GACTGCGTACCAATTC AAC-3'
MseI primer CGA:           5'-GATGAGTCCTGAGTAA CGA-3'
MseI primer CGAA:          5'-GATGAGTCCTGAGTAA CGAA-3'
MseI primer CGAC:          5'-GATGAGTCCTGAGTAA CGAC-3'
MseI primer CGAG:          5'-GATGAGTCCTGAGTAA CGAG-3'

MseI primer CGAT:          5'-GATGAGTCCTGAGTAA CGAT-3'
MseI primer CAAC:          5'-GATGAGTCCTGAGTAA CAAC-3'
PNA:                       5' -CCT GAG TAA CAA C

Figures 5A and 5B show DNA-fingerprints generated with radioactively-labeled *Eco*RI-primer 00AAC and *Msp*I-primers 00AAC, +4totAAC, 01AACA, 02AACC, 03AACG, 04AACT, +4totCAC, 00CAC, 01CACA, 02CACC, 03CACG, 04CACT on a template made with *Eco*RI and *Msp*I as the restriction enzymes. In the lanes +4totAAC and +4totCAC, primers 01AACA, 02AACC, 03AACG and 04AACT and primers 01CACA, 02CACC, 03CACG and 04CACT, respectively, were pooled in equimolar amounts and the total concentration of *Msp*I-primer in those reactions was kept the same as when only one *Msp*I-primer was used. AFLP reactions were preceeded by a +1+1 preamplification.

This figure illustrates that mispriming using +4 primers is not per se dependent on the enzyme combination.

MspI-adaptor 5'-GACGATGAGTCCTGAG-3'
3'-TACTCAGGACTCGC-5'

Preamplification primers:
EcoRI primer A GACTGCGTACCAATTC A
MspI primer A GATGAGTCCTGAGCGG A
MspI primer C GATGAGTCCTGAGCGG C

AFLP-primers
EcoRI primer 00AAC GACTGCGTACCAATTC AAC
MspI primer 00AAC GATGAGTCCTGAGCGG AAC
MspI primer 01AACA GATGAGTCCTGAGCGG AACA
MspI primer 02AACC GATGAGTCCTGAGCGG AACC
MspI primer 03AACG GATGAGTCCTGAGCGG AACG
MspI primer 04AACT GATGAGTCCTGAGCGG AACT
MspI primer 00CAC GATGAGTCCTGAGCGG CAC
MspI primer 01CACA GATGAGTCCTGAGCGG CACA
MspI primer 02CACC GATGAGTCCTGAGCGG CACC
MspI primer 03CACG GATGAGTCCTGAGCGG CACG
MspI primer 04CACT GATGAGTCCTGAGCGG CACT

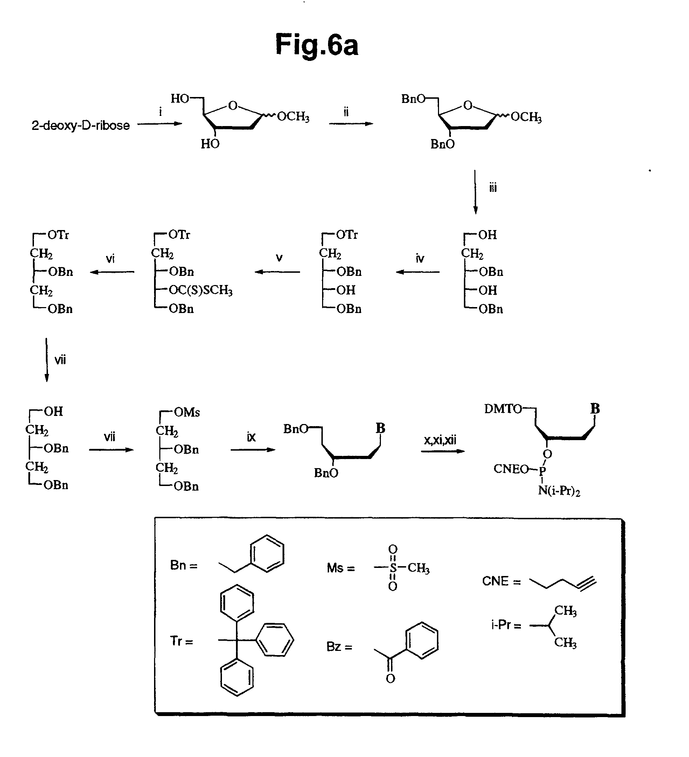

Figure 6A shows an exemplary synthesis of the acyclic nucleoside analogs useful in the invention. Reagentia and conditions: i, MeOH, AcCl; ii, DMF, NaH, BnBr; iii aq. HOAc; then NaBH₄, EtOH; iv, TrCl, C₅H₅N; v, THF, imidazole, NaH, CS₂, Mel; vi, Bu₃SnH, AIBN, toluene; vii, p-TsOH, MeOH, CH₂Cl₂; viii, MsCl, CH₂Cl₂, Et₃N; ix, NaH, B; x, cyclohexene, EtOH, Pd(OH)₂; xi, DMTCl, C₅H₅N; xii, CIP(OCNE)N(i-Pr)₂.

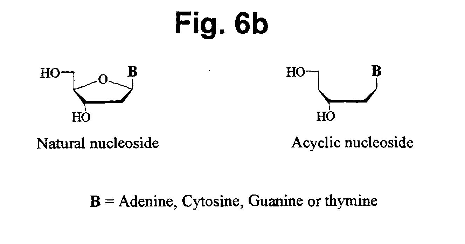

Figure 6B shows a natural nucleoside (left) and an acyclic nucleoside (right).

Figure 7 shows DNA fingerprints in which alternative nucleosides were used in *Mse*I-primers to cure mismatch amplification. All AFLP reactions were performed according to the standard procedures. AFLP reactions were preceeded by a +1+1 preamplification. Radioactively labeled *EcoR*I primer 00AAC was used as the *Eco*RI +3 primer in all AFLP reactions. Primer names are given in a way that they illustrate the differences between the primers used to generate the fingerprints.

**Fig 7A:** AFLP fingerprints after amplification with *EcoR*I primer 00AAC and *Mse*I primers 00TAA, 01TAA, 02TAA, 03TAA, 04TAA, 01TA(P), 02TA(P), 03TA(P), 04TA(P), 01T(P)A, 02T(P)A, 03T(P)A, 04T(P)A, respectively. The +3 extension of the *Mse*I-primers is CAC. +4 primers are supposed to generate fragments that represent a subset of the fragments present in the fingerprints generated with the +3 primer (00TAA). In the sequence of the primers, as shown below, P indicates the position of one 3-nitropyrrole residue

EcoRI primer 00AAC | GACTGCGTACCAATTC AAC
MseI primer 00TAA | GATGAGTCCTGAGTAACAC
MseI primer 01TAA | GATGAGTCCTGAGTAACACA
MseI primer 02TAA | GATGAGTCCTGAGTAACACC
MseI primer 03TAA | GATGAGTCCTGAGTAACACG
MseI primer 04TAA | GATGAGTCCTGAGTAACACT
MseI primer 01TA(P) | GATGAGTCCTGAGTA(P)CACA
MseI primer 02TA(P) | GATGAGTCCTGAGTA(P)CACC
MseI primer 03TA(P) | GATGAGTCCTGAGTA(P)CACG
MseI primer 04TA(P) | GATGAGTCCTGAGTA(P)CACT
MseI primer 01T(P)A | GATGAGTCCTGAGT(P)ACACA
MseI primer 02T(P)A | GATGAGTCCTGAGT(P)ACACC
MseI primer 03T(P)A | GATGAGTCCTGAGT(P)ACACG
MseI primer 04T(P)A | GATGAGTCCTGAGT(P)ACACT

**Fig. 7B:** AFLP fingerprints after amplification with with *EcoR*I primer 00AAC and *Mse*I primers TAA, TA(B), T(B)A, TA(PU) and T(PU)A, respectively. The +3 extension of the *Mse*I-primers is CAC. Only bands indicated with an asterix are non-mismatch bands. Primers TA(B) and T(B)A harbour one 2-deoxynebularine residue; primers TA(PU) and T(PU)A harbour one 2-aminopurine residu. In the sequence of the primers, as shown below, B indicates the position of the 2-deoxynebularine residue; PU indicates the position of the 2-aminopurine residue.

EcoRI primer 00AAC | GACTGCGTACCAATTC AAC
MseI primer TAA | GATGAGTCCTGAGTAA CACA
MseI primer TA(B) | GATGAGTCCTGAGTA(B)CACA
MseI primer T(B)A | GATGAGTCCTGAGT(B)ACACA
MseI primer TA(PU) | GATGAGTCCTGAGTA(PU)CACA
MseI primer T(PU)A | GATGAGTCCTGAGT(PU)ACACA

**Fig. 7C:** AFLP fingerprints after amplification with *EcoR*I primer 00AAC and *Mse*I primers 00TAA, 01TAA, 02TAA, 03TAA, 04TAA 00(altT)AA 01(altT)AA, 02(altT)AA, 03(altT)AA, 04(altT)AA, respectively. The +3 extension of the MseI-primers is CAC. +4 primers are supposed to generate fragments that represent a subset of the fragments present in the fingerprints generated with the +3 primer (00TAA). AltT primers harbour one nucleoside with an acyclic ribose, however with the standard base T. In the sequence of the primers, as shown below, (AltT) indicates the position of the altT residue.

EcoRI primer 00AAC GACTGCGTACCAATTC AAC
MseI primer 00TAA GATGAGTCCTGAGTAA CAC
MseI primer 01TAA GATGAGTCCTGAGTAA CACA
MseI primer 02TAA GATGAGTCCTGAGTAA CACC
MseI primer 03TAA GATGAGTCCTGAGTAA CACG
MseI primer 04TAA GATGAGTCCTGAGTAA CACT

MseI primer 00(altT)AA GATGAGTCCTGAG(altT)AA CAC
MseI primer 01(altT)AA GATGAGTCCTGAG(altT)AA CACA
MseI primer 02(altT)AA GATGAGTCCTGAG(altT)AA CACC
MseI primer 03(altT)AA GATGAGTCCTGAG(altT)AA CACG

MseI primer 04(altT)AA    GATGAGTCCTGAG(altT)AA CACT

**Fig. 7D:** AFLP fingerprints on twelve individuals of a Brassica F₂ population after amplification with *EcoR*I primer 00AAC and *Mse*I-primers 02(altT)AA, 02TAA and 00TAA, respectively. The +3 extension of the *Mse*I-primers is CAC. +4 primers are supposed to generate fragments that represent a subset of the fragments present in the fingerprints generated with the +3 primer (00TAA). 02(altT)AA harbours one nucleoside with an acyclic ribose, however with the standard base T. In the sequence of the primers, as shown below, (AltT) indicates the position of the altT residue.

EcoRI primer 00AAC | GACTGCGTACCAATTC AAC
MseI primer 02(altT)AA | GATGAGTCCTGAG(altT)AA CACC
MseI primer 02TAA | GATGAGTCCTGAGTAA CACC
MseI primer 00TAA | GATGAGTCCTGAGTAA CAC

**Fig. 7E:** AFLP fingerprints after amplification with *EcoR*I primer 00AAC and *Mse*I-primers TAA, T(I)A, TA(I), T(altA)A, respectively. The +4 extension of the *Mse*I-primers is AGCC. Only bands with indicated by an asterix are non-mismatch bands. Primers T(I)A and TA(I) harbour one inosine residue; T(altA)A harbours one nucleoside with an acyclic ribose, however with the standard base A. In the sequence of the primers, as shown below, I indicates the position of the inosine residue; (AltA) indicates the position of the altA residue.

EcoRI primer 00AAC | GACTGCGTACCAATTC AAC
MseI primer TAA | GATGAGTCCTGAGTAA AGCC
MseI primer T(I)A | GATGAGTCCTGAGT(I)A AGCC
MseI primer TA(I) | GATGAGTCCTGAGTA(I) AGCC
MseI primer T(altA)A | GATGAGTCCTGAGT(altA)A AGCC

**Fig. 7F:** AFLP fingerprints after amplification with *EcoR*I primer 00AAC and *Mse*I-primers 00TAA, 00TA(I), 00T(I)A, 01TAA, 01TA(I), 01T(I)A, 02TAA, 02TA(I), 02T(I)A, 03TAA, 03TA(I), 03T(I)A, 04TAA, 04TA(I), 04T(I)A, respectively. The +3 extension of the *Mse*I-primers is CGA. +4 primers are supposed to generate fragments that represent a subset of the fragments present in the fingerprints generated with the +3 primer (00TAA). Alternative primers harbour one inosine residue; in the sequence of the primers, as shown below, I indicates the position of the inosine residue.

EcoRI primer 00AAC | GACTGCGTACCAATTC AAC
MseI primer 00TAA | GATGAGTCCTGAGTAA CGA
MseI primer 00TA(I) | GATGAGTCCTGAGTA(I) CGA
MseI primer 00T(I)A | GATGAGTCCTGAGT(I)A CGA
MseI primer 01TAA | GATGAGTCCTGAGTAA CGAA
MseI primer 01TA(I) | GATGAGTCCTGAGTA(I) CGAA
MseI primer 01T(I)A | GATGAGTCCTGAGT(I)A CGAA
MseI primer 02TAA | GATGAGTCCTGAGTAA CGAC
MseI primer 02TA(I) | GATGAGTCCTGAGTA(I) CGAC
MseI primer 02T(I)A | GATGAGTCCTGAGT(I)A CGAC
MseI primer 03TAA | GATGAGTCCTGAGTAA CGAG
MseI primer 03TA(I) | GATGAGTCCTGAGTA(I) CGAG
MseI primer 03T(I)A | GATGAGTCCTGAGT(I)A CGAG
MseI primer 04TAA | GATGAGTCCTGAGTAA CGAT
MseI primer 04TA(I) | GATGAGTCCTGAGTA(I )CGAT
MseI primer 04T(I)A | GATGAGTCCTGAGT(I)A CGAT

**Fig. 7G:** AFLP fingerprints after amplification with *EcoR*I primer 00AAC and *Mse*I-primers 01TAA, 01TA(P), 01(altT)AA, 01TA(altA), 01TA(I), 01T(I)A, 01(II)A, 01T(II) (Fig.7G.1), 04TAA, 04TA(P), 04T(P)A, 04(altT)AA, 04TA(altA), 04TA(I), 04T(I)A, 04(II)A, 04T(II) (Fig.7G.2).

The +3 extension of the *Mse*I-primers is CAC. Only bands indicated with an asterix are non-mismatch bands. In the sequence of the primers, as shown below, P indicates the position of a 3-nitropyrrole residue. I indicates the position of one inosine residue; II indicates the position of two inosine residues. (AltA) indicates the position of an altA residue, (AltT) indicates the position of an altT residue.

EcoRI primer 00AAC GACTGCGTACCAATTC AAC
MseI primer 01TAA GATGAGTCCTGAGTAA CACA
MseI primer 01TA(P) GATGAGTCCTGAGTA(P)CACA
MseI primer 01(altT)AA GATGAGTCCTGAG(altT)AA CACA

MseI primer 01TA(altA) | GATGAGTCCTGAGTA(altA)CACA
MseI primer 01TA(I) | GATGAGTCCTGAGTA(I) CACA
MseI primer 01T(I)A | GATGAGTCCTGAGT(I)A CACA
MseI primer 01(II)A | GATGAGTCCTGAG(II)A CACA
MseI primer 01T(II) | GATGAGTCCTGAGT(II) CACA
MseI primer 04TAA | GATGAGTCCTGAGTAA CACT
MseI primer 04TA(P) | GATGAGTCCTGAGTA(P) CACT
MseI primer 04T(P)A | GATGAGTCCTGAGT(P)A CACT
MseI primer 04(altT)AA | GATGAGTCCTGAG(altT)AA CACT
MseI primer 04TA(altA) | GATGAGTCCTGAGTA(altA) CACT
MseI primer 04TA(I) | GATGAGTCCTGAGTA(I) CACT
MseI primer 04T(I)A | GATGAGTCCTGAGT(I)A CACT
MseI primer 04(II)A | GATGAGTCCTGAG(II)A CACT
MseI primer 04T(II) | GATGAGTCCTGAGT(II) CACT

### EXAMPLE 1: AFLP REACTIONS EMPLOYING STANDARD PRIMERS AND PRIMERS HARBOURING ALTERNATIVE NUCLEOSIDES ON DNA TEMPLATE OF BRASSICA PREPARED USING TWO RESTRICTION ENZYMES

In this example, AFLP employing either standard primers or primers harbouring alternative nucleosides is illustrated. AFLP was performed as described (Zabeau and Vos, European Patent Application, EP 0534858; Vos et al. 1995 Nucl. Acids. Res. Vol 23 No.21 pp 4407-4414) and is described briefly below.

### a. Isolation and modification of the DNA

Total Brassica (oilseed rape) DNA was isolated from young leaves using a modified CTAB procedure as described (Stewart C.J.jr. and Via L.E. 1993 BioTechniques 14, 748-750). In all AFLP reactions the restriction enzymes, *Eco*RI and *Mse*I were used for template preparation unless mentioned otherwise. Genomic DNA (0.5 µg) was incubated for 1 hour at 37 °C with 5 units *Eco*RI and 5 units *Mse*I in 40 µl 10 mM Tris.HAc pH 7.5, 10 mM MgAc, 50 mM KAc, 5 mM DTT, 50 ng/µl BSA. Subsequently, 10 µl of a solution containing 5 pMol *Eco*RI-adapters, 50 pMol MseI-adapters, 1 unit T4 DNA-ligase, 1 mM ATP in 10 mM Tris.HAc pH 7.5, 10 mM MgAc, 50 mM KAc, 5 mM DTT, 50 ng/µl BSA was added, and the incubation was continued for 3 hours at 37°C.

The structure of the *Eco*RI-adapter was:

5'-CTCGTAGACTGCGTACC
CATCTGACGCATGGTTAA-5'

The structure of the *Mse*I-adapter was:

5'-GACGATGAGTCCTGAG
TACTCAGGACTCAT-5'

Adapters were prepared by adding equimolar amounts of both strands; adapters were not phosphorylated. After ligation, the reaction mixture was diluted to 500 µl with 10 mM Tris.HCl, 0.1 mM EDTA pH 8.0, and stored at -20°C. The diluted reaction mixture is further referred to as template DNA.

When other enzyme combinations are used different adaptors are used that are compatible with the cohesive ends generated by those enzymes, however the core sequence of the adapters may remain the same as used in the *Eco*RI/*Mse*I enzyme combination. It is important, however, when 2 enzymes are used that the two core sequences of the adaptors that fit the two different sticky ends are different. Primers are adapted accordingly.

### b. AFLP preamplification reactions

The primers used for the AFLP preamplifcations (+1-primers) are depicted below:

EcoRI-primers: 5'-GACTGCGTACCAATTCN-3'
MseI-primers:  5'-GATGAGTCCTGAGTAAN-3'

AFLP primers consist of a constant part and a variable part. The N in the primers indicate that this part of the primers was variable. There are 4 possible +1 primers for each of the *Eco*RI and *Mse*I +1 primers with extensions A, C, G, or T. They are employed in the following way: when AFLP reaction primers (+3 or +4) start with an A, C, G, or T, respectively, as the first selective nucleoside, the preamplification primers also have an A, C, G or T, respectively, as the selective nucleoside.

In AFLP preamplification reactions 20 µl reaction mixtures were prepared containing 30 ng of both the *Eco*RI- and the *Mse*I-primer, 5 µl template-DNA, 0.4 units *Taq*-polymerase, 10 mM Tris.HCl pH 8.3, 1.5 mM MgCl₂, 50 mM KCl, 0.2 mM of all 4 dNTPs. Primers were not radioactively labeled. AFLP preamplification reactions were performed for 20 cycles using the following cycle profile: a 30 seconds DNA denaturation step at 94 °C, a 30 seconds annealing step at 56 °C and a 1 minute extension step at 72 °C. All amplification reactions were performed in a PE-9600 thermocycler (Perkin Elmer Corp., Norwalk, CT, USA). After this preamplification step the reaction mixtures were diluted 20-fold with 10 mM Tris.HCl, 0.1 mM EDTA pH 8.0 and referred to as preamplified template for the second amplification reaction (the AFLP-reaction).

### c. AFLP reactions

The standard primers used for AFLP amplification reactions are depicted below:

EcoRI-primers: 5'-GACTGCGTACCAATTCNNN-3'

MseI-primers: 5'-GATGAGTCCTGAGTAANNN-3'
5'-GATGAGTCCTGAGTAANNNN-3'

The N's in the primers indicate that this part of the primers was variable: There are in principle 64 possible primers with three selective nucleosides (NNN) and 256 possible primers with four selective nucleosides (NNNN).

Alternative primers basically have the same sequence, however, one or more of the nucleosides in any position in the primer can be replaced by one or more alternative nucleosides.

The AFLP reactions were performed in the following way: AFLP reactions employed either a radio-actively labeled *Eco*RI-primer and a non-labeled *Mse*I-primer or a radio-actively labelled *Mse*I-primer and a non-labelled *Eco*RI-primer. Primers were end-labelled using (γ-³³P)ATP and T4 polynucleotide kinase. The labelling reactions were performed in 50 µl 25 mM Tris.HCl pH 7.5, 10 mM MgCl₂, 5 mM DTT, 0.5 mM spermidine.3HCl using 500 ng oligonucleotide primer, 100 µCi (γ-³³P)ATP and 10 units T4 polynucleotide kinase. For AFLP analysis 20 µl reaction mixtures were prepared containing 5 ng labeled primer (0.5 µl from the labelling reaction mixture), 30 ng unlabeled primer, 5 µl preamplified template-DNA, 0.4 units Taq-polymerase, 10 mM Tris.HCl pH 8.3, 1.5 mM MgCl₂, 50 mM KCl, 0.2 mM of all 4 dNTPs. AFLP reactions were performed using the following cycle profile: a 30 seconds DNA denaturation step at 94 °C, a 30 seconds annealing step (see below), and a 1 minute extension step at 72 °C. The annealing temperature in the first cycle was 65 °C, was subsequently reduced each cycle by 0.7 °C for the next 12 cycles, and was continued at 56 °C for the remaining 23 cycles. All AFLP reactions were performed in a PE-9600 thermocycler (Perkin Elmer Corp., Norwalk, CT, USA).

### d. Gel analysis of AFLP reaction products

After amplification, reaction products were mixed with an equal volume (20 µl) of formamide dye (98% formamide, 10 mM EDTA pH 8.0, and bromo phenol blue and xylene cyanol as tracking dyes). The resulting mixtures were heated for 3 minutes at 90 °C, and then quickly cooled on ice. 2 µl of each sample was loaded on a 5% denaturing (sequencing) polyacrylamide gel (Maxam and Gilbert, 1980, Methods in Enzymology 65, 499-560). The gel matrix was prepared using 5% acrylamide, 0.25% methylene bisacryl, 7.5 M urea in 50 mM Tris/50 mM Boric acid/1 mM EDTA. To 100 ml of gel solution 500 µl of 10% APS and 100 µl TEMED was added and gels were cast using a SequiGen 38 x 50 cm gel apparatus (Biorad Laboratories Inc., Hercules, CA, USA). Sharktooth combs were used to give 97 lanes on the SequiGen gel units. 100 mM Tris/100 mM Boric acid/2 mM EDTA was used as running buffer. Electrophoresis was performed at constant power, 110 Watts, for approximately 2 hours. After electrophoresis, gels were fixed for 30 minutes in 10% acetic acid, dried on the glass plates and exposed to Fuji phospho image screens for 16 hrs. Fingerprint patterns were visualized using a Fuji BAS-2000 phospho image analysis system (Fuji Photo Film Company Ltd, Japan).

Figure 2 and Figure 5 illustrate, that, following the standard AFLP procedure, +4 primers do not generate a subset of the fragments generated by the corresponding +3 primer, however, many contaminating bands are present in the fingerprints. Therefore, the four +4 primers with A, C, G, and T as the fourth selective nucleoside do not together generate the corresponding +3 fingerprint.

Figure 7 illustrates the use of *MseI*-primers with alternative nucleosides to cure the mismatch amplification as illustrated in Figure 1.

### EXAMPLE 2: AFLP REACTIONS PERFORMED ON THE SAME TEMPLATE USING DIFFERENT +4 PRIMERS GENERATE THE SAME PRODUCTS

In this example it is illustrated that different +4 primers do in part generate similar products and that many if not all mispriming products are the result of mispriming on one position.

AFLP preamplification reactions were performed using the following primers:

### Preamplification primers:

EcoRI primer A:    5'-GACTGCGTACCAATTC A
MseI primer  C:    5'-GATGAGTCCTGAGTAA C

AFLP reactions were performed using the following primers: AFLP reaction primers:

EcoRI primer 00AAC:       5'-GACTGCGTACCAATTCAAC-3'

MseI primer CGA:   5'-GATGAGTCCTGAGTAA CGA-3'
MseI primer CGAA:  5'-GATGAGTCCTGAGTAA CGAA-3'
MseI primer CGCA:  5'-GATGAGTCCTGAGTAA CGCA-3'
MseI primer CGGA:  5'-GATGAGTCCTGAGTAA CGGA-3'
MseI primer CGTA:  5'-GATGAGTCCTGAGTAA CGTA-3'
MseI primer CAAA:  5'-GATGAGTCCTGAGTAA CAAA-3'
MseI primer CCAA:  5'-GATGAGTCCTGAGTAA CCAA-3'
MseI primer CTAA:  5'-GATGAGTCCTGAGTAA CTAA-3'

MseI primer CGAC:  5'-GATGAGTCCTGAGTAA CGAC-3'
MseI primer CGCC:  5'-GATGAGTCCTGAGTAA CGCC-3'
MseI primer CGGC:  5'-GATGAGTCCTGAGTAA CGGC-3'
MseI primer CGTC:  5'-GATGAGTCCTGAGTAA CGTC-3'
MseI primer CAAC:  5'-GATGAGTCCTGAGTAA CAAC-3'
MseI primer CCAC:  5'-GATGAGTCCTGAGTAA CCAC-3'
MseI primer CTAC:  5'-GATGAGTCCTGAGTAA CTAC-3'

MseI primer CGAG:  5'-GATGAGTCCTGAGTAA CGAG-3'
MseI primer CGCG:  5'-GATGAGTCCTGAGTAA CGCG-3'
MseI primer CGGG:  5'-GATGAGTCCTGAGTAA CGGG-3'
MseI primer CGTG:  5'-GATGAGTCCTGAGTAA CGTG-3'
MseI primer CAAG:  5'-GATGAGTCCTGAGTAA CAAG-3'
MseI primer CCAG:  5'-GATGAGTCCTGAGTAA CCAG-3'
MseI primer CTAG:  5'-GATGAGTCCTGAGTAA CTAG-3'

MseI primer CGAT:  5'-GATGAGTCCTGAGTAA CGAT-3'
MseI primer CGCT:  5'-GATGAGTCCTGAGTAA CGCT-3'
MseI primer CGGT:  5'-GATGAGTCCTGAGTAA CGGT-3'
MseI primer CGTT:  5'-GATGAGTCCTGAGTAA CGTT-3'
MseI primer CAAT:  5'-GATGAGTCCTGAGTAA CAAT-3'
MseI primer CCAT:  5'-GATGAGTCCTGAGTAA CCAT-3'
MseI primer CTAT:  5'-GATGAGTCCTGAGTAA CTAT-3'

Primers were chosen to have one nucleoside different with one of the primers 
MseI primer CGAA:   5′-GATGAGTCCTGAGTAA CGAA-3′
MseI primer CGAC:   5′-GATGAGTCCTGAGTAA CGAC-3′
MseI primer CGAG:   5′-GATGAGTCCTGAGTAA CGAG-3′
MseI primer CGAT:   5′-GATGAGTCCTGAGTAA CGAT-3′
 that theoretically together should only generate fragments present in the fingerprint with *Mse*I-primer CGA. It was shown that the *Mse*I-primers listed above in combination with *Eco*RI primer 00AAC are able to generate similar bands as the *Mse*I primers CGAA, CGAC, CGAG, CGAT (Fig.3).

By identifying most of the mismatch templates that could explain mismatch bands in fingerprints generated with *Mse*I primers CGAA, CGAC, CGAG and CGAT, it was concluded that mismatch mostly occurs at one position in template molecules.

Similar experiments were performed with *Eco*RI primer 00AAC and *Mse*I-primers AGC, CAG, CCT, and CTG, respectively; and the +4 primers deduced from those +3 primers. Fingerprints were again compared with fingerprints that were generated with primers having one mismatch with +4 primers at the +2 and+3 positions, leading to the same conclusion that different +4 primers do in part generate similar products and that many if not all mispriming products are the result of mispriming on one position.

MseI primer AGC:    5'-GATGAGTCCTGAGTAA AGC-3'
MseI primer CAG:    5'-GATGAGTCCTGAGTAA CAG-3'
MseI primer CCT:    5'-GATGAGTCCTGAGTAA CCT-3'
MseI primer CTG:    5'-GATGAGTCCTGAGTAA CTG-3'

### EXAMPLE 3: USE OF PNA TO PREVENT AMPLIFICATION OF MISPRIMING PRODUCTS

In this example it is illustrated that amplification of products that are the result of mispriming can be prevented by adding specific Peptide Nucleic Acid (PNA) molecules to the reaction as long as the target selective nucleosides are known. Here, it is demonstrated that some mispriming products have indeed the sequence as deduced in example 2 (Fig.3) since amplification is prevented by a specific PNA (Fig. 4).

AFLP preamplification reactions were performed using the following primers:

### Preamplification primers:

EcoRI-primer A:    5'-GACTGCGTACCAATTC A
MseI-primer C:     5'-GATGAGTCCTGAGTAA C

AFLP reactions were performed using the following primers:

### AFLP reaction primers:

EcoRI primer 00AAC:        5'-GACTGCGTACCAATTCAAC-3'

MseI primer CAAC:        5'-GATGAGTCCTGAGTAACAAC-3'

MseI primer CGA:          5'-GATGAGTCCTGAGTAACGA-3'
MseI primer CGAC:         5'-GATGAGTCCTGAGTAACGAC-3'

PNA of the following sequence was obtained from PerSeptive Biosystems, Inc, Framingham, MA:

5′ H- CCT GAG TAA CAA C - CONH2

PNA in quantities of 0 and 35 pmoles was added to AFLP reactions using *Eco*RI primer 00AAC and *Mse*I primers, CGA, +4tot, CGAC and CAAC, respectively. Results are shown in Fig. 4. The PNA oligo specifically blocks template amplified with *Mse*I primer CAAC. The fingerprint shows that indeed part of this template is also amplified with *Mse*I primer CGAC and +4tot since similar bands are blocked in fingerprints that represent AFLP reactions including PNA.

### EXAMPLE 4: USE OF ALTERNATIVE NUCLEOSIDES IN PRIMERS AS A STRATEGY TO INHIBIT OR PREVENT THE AMPLIFICATION OF MISMATCH BANDS.

Alternative nucleosides with acyclic riboses were synthesized at the Department of Organic Chemistry, Gorlaeus laboratoria, Rijksuniversiteit Leiden, The Netherlands (fig. 6), all other alternative nucleosides were commercially available (Glen Research, Sterling, Virginia). All alternative nucleotides were incorporated in primers following standard procedures. Alternative nucleotides were incorporated in several different positions in primers. Oligonucleotides were generally used as adapters and primers for AFLP without further purification, except for primers with nucleosides with acyclic riboses that were HPLC purified after synthesis.

Figure 7 illustrates the use of *Mse*I-primers with alternative nucleosides to cure mismatch amplification.

## Claims

1. Use of a selective primer containing one or more selective nucleotides at its 3' end in selective restriction fragment amplification (AFLP), whereby the primer contains one or more alternative nucleosides in a position 2-10 bases from the 3'-end of the primer sequence, whereby the alternative nucleoside is a nucleoside having an alteration in the base nucleus, the sugar residue, the bond between the base nucleus and the sugar residue, the linkage of the nucleoside to other nucleosides in the primer sequence or any combination thereof, compared to the naturally occurring nucleosides A, T, C G and U, and whereby in a primer/template duplex the alternative nucleoside binds less strongly to a nucleoside in an opposite position in the template as compared to a naturally occuring nucleoside complementary to the nucleoside in the template.

2. Use according to claim 1, in which the one or more alternative nucleosides are present in a position 2-8 bases from the 3'-end of the primer sequence.

3. Use according to any of the preceding claims, in which the primer contains less than three alternative nucleoside(s).

4. Use according to any of the preceding claims, in which the primer contains 2-8 selective nucleotides.

5. Use according to any of the preceding claims, in which the one or more alternative nucleosides are present in either or both of the variable region of the primer sequence, and the first four base positions adjacent to the variable region.

6. Use according to any of claims 1-5, in which the alternative nucleoside is a-nucleoside in which, compared to the naturally occuring nucleosides A, T, C and G, the purine or pyrimidine base nucleus has been replaced by a corresponding aza- or deaza-analog thereof, and/or a substituted analog thereof.

7. Use according to any of claims 1-6, in which the alternative nucleoside is a nucleoside in which, compared to the naturally occuring nucleosides A, T, C and G, the purine or pyrimidine base nucleus has been replaced by a nitro-substituted heterocyclic group.

8. Use according to claim 7, in which the nitro-substituted heterocyclic group is a 3-nitro-substituted nitropyrrole group, a 3-nitro-substituted diazole group or a 3-nitro-substituted triazole group.

9. Use according to any of claims 1-6, in which the alternative nucleoside is a nucleoside in which, compared to the naturally occurring nucleosides A, T, C and G, the sugar residue has been replaced by an alicylic sugar residu.

10. Use according to any of claims 1-6, in which the primer contains one or more peptide nucleic acids as the alternative nucleoside(s).

11. Use according to any of claims 1-6, in which the primer is derived from an AFLP primer known per se, by replacing one or more of the native nucleotides A, T, C or C in the sequence of said known primer with either a destabilising nucleoside analog thereof, or by a non-selective nucleoside.

12. Use according to claim 11, in which the primer is derived from a known AFLP primer, by replacing one or more of the native nucleotides A, T, C or C in the sequence of said known primer with a nucleoside analog based on inosine or the inosine nucleus.

13. Use according to any of the preceding claims, in which the primer is derived from a known AFLP primer, by replacing one or more of the native nucleotides A, T, C or C in the sequence of said known primer with a detectable and/or distinguishable nucleoside analog.

14. Method for carrying out AFLP, in which a known AFLP primer is replaced by a primer as defined in claim 1, and the amplification and optionally the detection of the amplified fragments are carried out in a manner known per se.

15. Method for carrying out AFLP, in which a known AFLP primer is replaced by a primer as defined in claim 1, whereby the amplification is carried out in a manner known per se, and whereby the amplified fragments are detected, identified and/or visualised by a detection technique based upon the physical, chemical and/or biological properties of the one or more detectable alternative nucleosides, or of the nucleic acid strand in which they have been incorporated, whereby the detection technique is not gel-electrophoresis.

16. Kit for AFLP, comprising a primer as defined in claim 1, and a double stranded synthetic oligonucleotide adapter, whereby one end of the adapter is compatible for ligation onto one or both ends of a restriction fragment, whereby the primer contains a region that is complementary to at least part of the sequence of the adapter, and whereby the kit optionally comprises further components known per se for AFLP-kits.

## Patentansprüche

1. verwendung eines selektiven Primers, enthaltend ein oder mehrere selektive Nukleotide an seinem 3'-Ende in selektiver Restriktions-Fragment-Amplifikation (AFLP), wobei der Primer ein oder mehrere alternative Nukleoside in einer Position 2-10 Basen von dem 3'-Ende der Primersequenz enthält, wobei das alternative Nukleosid ein Nukleosid mit einer Veränderung in dem Basenkern, dem Zuckerrest, der Bindung zwischen dem Basenkern und dem Zuckerrest, der Verknüpfung des Nukleosids mit anderen Nukleosiden in der Primersequenz oder einer beliebigen Kombination davon, verglichen mit den natürlich vorkommenden Nukleosiden A, T, C, G und U ist, und wobei in einem Primer/Template-Duplex das alternative Nukleosid weniger stark an ein Nukleosid in einer gegenüberliegenden Position in dem Template bindet verglichen mit einem natürlich vorkommenden Nukleosid, welches komplementär zu dem Nukleosid in dem Template ist.

2. Verwendung nach Anspruch 1, in welcher das/die eine oder mehreren alternative Nukleoside in einer Position 2-8 Basen vom 3'-Ende der Primersequenz vorhanden sind.

3. Verwendung nach einem beliebigen der vorangehenden Ansprüche, in welcher der Primet weniger als 3 alternative(s) Nukleosid(e) enthält.

4. Verwendung nach einem beliebigen der vorangehenden Ansprüche, in welcher der Primer 2-8 selektive Nukleotide enthält.

5. Verwendung nach einem beliebigen der vorangehenden Ansprüche, in welcher das/die eine oder mehreren altemative(n) Nukleosid(e) in einer von beiden oder beiden variablen Regionen der Primersequenz vorhanden sind und die ersten 4 Basenpositionen benachbart zu der variablen Region.

6. Verwendung nach einem beliebigen der Ansprüche 1-5, in welcher das alternative Nukleosid ein Nukleosid ist, in welchem, verglichen mit den natürlich vorkommenden Nukleosiden A, T, C, und G der Purin- oder Pyrimidinbasenkem durch ein entsprechendes Aza- oder Deazaanalogon davon ersetzt wurde und/oder ein substituiertes Analogon davon.

7. Verwendung nach einem beliebigen der Ansprüche 1-6, in welcher das alternative Nukleosid ein Nuklcosid ist, in welchem, verglichen mit den natürlich vorkommenden Nukleosiden A, T, C und G der Purin- oder Pyrimidinbasenkern durch eine Nitro-substituierte heterozyklische Gruppe ersetzt worden ist.

8. Verwendung nach Ansprach 7, in welcher die Nitro-substituierte heterozyklische Gruppe eine 3-Nitro-substituierte Nitropyrrolgruppe, eine 3-Nitro-substituierte Diazolgruppe oder eine 3-Nitro-substituierte Triazolgruppe ist.

9. Verwendung nach einem beliebigen der Ansprüche 1-6, in welcher das alternative Nukleosid ein Nukleosid ist, in welchem verglichen mit den natürlich vorkommenden Nukleosiden A, T, C und G der Zuckerrest durch einen alizyklischen Zuckerrest ersetzt worden ist.

10. Verwendung nach einem beliebigen der Ansprüche 1-6, in welcher der Primer eine oder mehrere Peptidnukleinsäuren als das/die alternative(n) Nukleosid(e) enthält.

11. Verwendung nach einem beliebigen der Ansprüche 1-6, in welcher der Primer von einem an sich bekannten AFLP-Primer abgeleitet ist, durch Ersetzen eines oder mehrerer der nativen Nukleotide A, T, C oder C, in der Sequenz des genannten bekannten Primers, mit entweder einem destabilisierenden Nukleosidanalogon davon oder durch ein nicht-selektives Nukleosid.

12. Verwendung nach Anspruch 11, in welcher der Primer von einem bekannten AFLP-Primer abgeleitet ist, durch Ersetzen von einem oder mehreren der nativen Nukleotide A, T, C oder C in der Sequenz des genannten bekannten Primers, mit einem Nukleosidanalogon, das auf Inosin oder dem Inosinkern basiert.

13. Verwendung nach. einem beliebigen der vorangehenden Ansprüche, in welcher der Primer von einem bekannten AFLP-Primer abgeleitet ist, durch Ersetzen eines oder mehrerer der nativen Nukleotide A, T, C oder C in der Sequenz des genannten bekannten Primers mit einem detektierbaren und/oder unterscheidbaren Nukleosidanalogon.

14. Verfahren zur Durchführung von AFLP, in welchem ein bekannter AFLP-Primer durch einen wie in Anspruch 1 definierten Primer ersetzt wird und die Amplifikation und wahlweise die Detektion der amplifizierten Fragmente in einer an sich bekannten Weise durchgeführt wird.

15. Verfahren zur Durchführung von AFLP, in welchem ein bekannter AFLP-Primer durch einen wie in Anspruch 1 definierten Primer ersetzt wird, wobei die Amplifikation in einer an sich bekannten Weise durchgeführt wird, und wobei die amplifizierten Fragmente detektiert, identifiziert und/oder sichtbar gemacht werden durch eine Detektionstechnik, die auf den physikalischen, chemischen und/oder biologischen Eigenschaften von dem/den einen oder mehreren detektierbaren alternativen Nukleosiden oder von dem Nukleinsäurestrang, in welchen sie eingebaut worden sind, basieren, wobei die Detektionstechnik keine Gelelektrophorese ist.

16. Kit für AFLP, umfassend einen wie in Anspruch 1 definierten Primer und einen doppelsträngigen synthetischen Oligonukleotidadapter, wobei ein Ende des Adapters kompatibel zur Ligation an eines der beiden oder beide Enden eines Restriktionsfragments ist, wobei der Primer eine Region enthält, die komplementär zu mindestens einem Teil der Sequenz des Adapters ist, und wobei das Kit wahlweise weitere an sich bekannte Komponenten für AFLP-Kits umfasst.

## Revendications

1. Utilisation d'une amorce sélective contenant un ou plusieurs nucléotides sélectifs en son extrémité 3' dans une amplification de fragments par restriction sélective (AFLP), l'amorce contenant un ou plusieurs nucléosides de remplacement dans une position entre 2 et 10 bases de l'extrémité 3' de la séquence de l'amorce, le nucléoside de remplacement étant un nucléoside présentant une altération dans le noyau basique, le radical de sucre, la liaison entre le noyau basique et le radical de sucre, le lien du nucléoside à d'autres nucléosides dans la séquence de l'amorce ou une quelconque combinaison de celles-ci, par rapport aux nucléosides naturels A, T, C, G, et U et, dans un duplex amorce/matrice, le nucléoside de remplacement se liant moins fortement avec un nucléoside dans la position opposée dans la matrice par rapport à un nucléoside naturel complémentaire au nucléoside dans la matrice.

2. Utilisation selon la revendication 1, dans laquelle un ou plusieurs nucléosides de remplacement sont présents dans une position entre 2 et 8 bases de l'extrémité 3' de la séquence de l'amorce.

3. Utilisation selon l'une quelconque des revendications précédentes, dans laquelle l'amorce contient moins de trois nucléosides de remplacement.

4. Utilisation selon l'une quelconque des revendications précédentes dans laquelle l'amorce contient 2 à 8 nucléotides sélectifs.

5. Utilisation selon l'une quelconque des revendications précédentes, dans laquelle le ou les nucléosides de remplacement sont présents dans un ou les deux sites variables de la séquence de l'amorce et les quatre premières positions des bases adjacentes au site variable.

6. Utilisation selon l'une quelconque des revendications 1 à 5, dans laquelle le nucléoside de remplacement est un nucléoside dans lequel, par rapport aux nucléosides naturels A, T, C et G, le noyau basique purine ou pyrimidine a été remplacé par un analogue aza ou déaza de celui-ci et/ou un analogue substitué de ceux-ci.

7. Utilisation selon l'une quelconque des revendications 1 à 6, dans laquelle le nucléoside de remplacement est un nucléoside dans lequel, par rapport aux nucléosides naturels A, T, C et G, le noyau basique purine ou pyrimidine a été remplacé par un groupe hétérocyclique dont un groupe nitro a été substitué.

8. Utilisation selon la revendication 7, dans laquelle le groupe hétérocyclique ayant un groupe nitro substitué est un groupe nitropyrrole dont le groupe nitro de la 3ème position est substitué, un groupe diazole dont le groupe nitro de la 3éme position est substitué, ou un groupe triazole dont le groupe nitro de la 3ème position est substitué.

9. Utilisation selon l'une quelconque des revendications 1 à 6, dans laquelle le nucléoside de remplacement est un nucléoside dans lequel, par rapport aux nucléosides naturels A, T, C et G, le radical de sucre a été remplacé par un radical de sucre alicylique.

10. Utilisation selon l'une quelconque des revendications 1 à 6, dans laquelle l'amorce contient un ou plusieurs acides nucléiques peptidiques comme nucléosides de remplacement.

11. Utilisation selon l'une quelconque des revendications 1 à 6, dans laquelle l'amorce est dérivée d'une amorce AFLP connue en soi, en remplaçant un ou plusieurs des nucléosides d'origine A, T, C ou G dans la séquence de ladite amorce connue par un nucléoside de déstabilisation analogue à celui-ci ou par un nucléoside non sélectif.

12. Utilisation selon la revendication 11, dans laquelle l'amorce est dérivée d'une amorce AFLP connue en soi, en remplaçant un ou plusieurs des nucléosides d'origine A, T, C ou G dans la séquence de ladite amorce connue par un analogue de nucléoside basé sur l'inosine ou le noyau inosine.

13. Utilisation selon l'une quelconque des revendications précédentes, dans laquelle l'amorce est dérivée d'une amorce AFLP connue, en remplaçant un ou plusieurs des nucléotides d'origine A, T, C ou G dans la séquence de ladite amorce connue par un analogue de nucléoside détectable et/ou distinguable.

14. Procédé pour effectuer la AFLP dans lequel une amorce AFLP connue est remplacée par une amorce telle que définie dans la revendication 1 et l'amplification et éventuellement la détection des fragments amplifiés sont effectuées d'une manière connue en soi.

15. Procédé pour effectuer la AFLP, dans lequel une amorce AFLP connue est remplacée par une amorce telle que définie dans la revendiration 1, l'amplification étant réalisée d'une manière connue en soi et les fragments amplifiés étant détectés, identifiés et/ou visualisés par une technique de détection basée sur les propriétés physiques, chimiques et/ou biologiques d'un ou de plusieurs nucléosides de remplacement détectables ou du brin d'acide nucléique dans lequel ils ont été incorporés, la technique de détection n'étant pas une électrophorése sur gel.

16. Kit pour l'AFLP, comprenant une amorce telle que définie dans la revendication 1 et un adaptateur synthétique oligonuctéotide à double brin, une extrémité de l'adaptateur étant compatible pour la ligation sur une ou les deux extrémités d'un fragment de restriction, l'amorce contenant un site qui est complémentaire à au moins une partie de la séquence de l'adaptateur et le kit contenant éventuellement d'autres composants connus en soi pour les kits AFLP.
